# EUROPEAN PATENT APPLICATION

(11) **EP 4 564 359 A1**
(43) Date of publication of application: **04.06.2025**
(21) Application number: 24210524.5
(22) Date of filing: 04.11.2024
(51) Int. Cl.: G16H 10/60, A61B 5/00, G06N 20/00, G16H 20/00, G16H 40/60, G16H 50/20, G16H 50/70

(54) **SYSTEM FOR DIAGNOSIS DECISION SUPPORT BY AN AI ASSISTED AND OPTIMIZED CARE ASSISTANCE TOOL, AND ASSOCIATED METHOD**

(30) Priority: 29.11.2023 US 202318523540
(71) Applicant: GE Precision Healthcare LLC, Waukesha, WI 53188 (US)
(72) Inventor: MISHRA, Isani, 560066 Bangalore (IN); VESTO, Guy Robert, Kildeer, 60047 (US); JANSSEN, Brian Dale, Brookfield, 53045 (US)
(74) Representative: Kilburn & Strode LLP

(57) **Abstract**

A system for assisting in care of a diagnosed medical patient. The system including an edge device, a plurality of patient monitoring devices, and a database including medical information. The edge device being configured to obtain a care plan for a patient; obtain a care recommendation by inputting a diagnosis and one or more of medical information corresponding to the patient, monitoring data corresponding to the patient, and medical facility inventory information into a patient care model; compare the care recommendation to the care plan to obtain a care insight, the care insight including recommended care that is not included in the care plan; output information corresponding to the care insight, and responsive to a user input selecting the care insight through the human-machine interface, revising the care plan based on the care insight.

## Description

### FIELD

Embodiments of the subject matter disclosed herein relate to caring for patients in a healthcare environment, and in particular to providing assistance in caring for patients using an artificial intelligence (AI) assisted guidance tool.

### BACKGROUND

Health care providers (e.g., physicians, nurse practitioners, and physician assistants) utilize a thought process, referred to as differential diagnosis, in diagnosing a patient. Differential diagnosis involves determining multiple possible diagnoses of the patient based on the available patient health data and symptoms of the patient. The physician then refines down the differential diagnosis, by performing additional diagnostic tests or assessing additional health data of the patient until he/she is confident that he/she has identified the correct diagnosis. Gathering the health data of the patient for differential diagnosis may involve the care provider searching for medical information regarding the patient in multiple, different hospital (or clinic) information systems (e.g., referred to as "Healthcare Information Technology (HCIT) systems"). This process may be time consuming and error prone, especially when the patient has an extensive medical history. Additionally, the care giver may need to consult available medical standards and guidelines, or recall these from memory, when performing the differential diagnosis and deciding how to treat the patient. This may also be time consuming and/or result in the care provider missing a possible diagnosis.

### BRIEF DESCRIPTION

According to an aspect of the disclosure, a medical system may include one or more patient monitoring devices, each patient monitoring device being configured to generate patient monitoring data by monitoring a physiological parameter of a patient; at least one database storing medical information corresponding to the patient; and an electronic device including a power source; a communication interface configured to communicate with the one or more patient monitoring devices and the at least one database over a network; a human-machine interface configured to provide information to a user and obtain information from the user; a memory configured to store instructions; and at least one processor configured to execute the instructions to: obtain, through the communication interface, a care plan for the patient; obtain a care recommendation by inputting a diagnosis and one or more of medical information corresponding to the patient, monitoring data corresponding to the patient, and medical facility inventory information into a patient care model; compare the care recommendation to the care plan to obtain a care insight, the care insight including recommended care that is not included in the care plan; output, through the human-machine interface, information corresponding to the care insight, and responsive to a user input selecting the care insight through the human-machine interface, revise the care plan based on the care insight.

According to another aspect of the disclosure, a method of patient care assistance may include: obtaining a care plan for a patient; obtaining a care recommendation by inputting a diagnosis and one or more of medical information corresponding to the patient, monitoring data corresponding to the patient, and medical facility inventory information into a patient care model; comparing the care recommendation to the care plan to obtain a care insight, the care insight including recommended care that is not included in the care plan; outputting information corresponding to the care insight, and responsive to a user input selecting the care insight through the human-machine interface, revising the care plan based on the care insight.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 schematically shows an example healthcare provider assistance system, according to an embodiment.
FIG. 2 is an input/output diagram of the DAM assistant, according to an embodiment
FIG. 3A is a chart of a differential diagnosis list and corresponding predicted probabilities over a 12 hour period, according to an embodiment.
FIG. 3B is a chart of the differential diagnosis list, along with clinical assessments, parameters monitored, and test performed over the 12 hour period, according to an example.
FIG. 3C is a graph of changes in the predicted probabilities of the diagnoses on the differential diagnosis list over the 12 hour period, according to an embodiment.
FIG. 4A is a table of data produced by the DAM assistant at a point in time corresponding to hour 0 of the example of FIGs. 3A though 3C, according to an embodiment.
FIG. 4B is a table of data produced by the DAM assistant at a point in time corresponding to hour 4 of the example of FIGs. 3A though 3C, according to an embodiment.
FIGS. 5A-5F show outputs of a chatbot interface of the DAM assistant, according to an embodiment.
FIG. 6 is a diagram of an electronic device on which the DAM assistant deployed, partially deployed, operated, or accessed, according to an embodiment.
FIG. 7 is a diagram of a network environment on which the DAM assistant deployed, partially deployed, operated, or accessed, according to an embodiment.
FIG. 8 is a flowchart of a method 800 of diagnosing and monitoring a patient, according to an embodiment.
FIG. 9 is a flow chart of a process 900 for training an AI model to generate a differential diagnosis list and corresponding predicted probabilities, according to an embodiment.
FIG. 10 is an input/output diagram of the patient care assistant, according to an embodiment.
FIGS. 11A-D show an example of outputs of the care assistant, according to an embodiment.
FIGS. 12A-12G show a chatbot interface for the care assistant, according to an embodiment.
FIGS. 13A and 13B are flowcharts showing a care assistance method 1300, according to an embodiment.

### DETAILED DESCRIPTION

The following description relates to various embodiments of a healthcare provider assistance system that compiles patient health data from a plurality of sources, processes the data to generate meaningful information, and offers guidance to a healthcare provider. The disclosed system may be implemented using a diagnosis and monitoring (DAM) assistant. The DAM assistant may provide a dynamic differential diagnosis list that includes one or more diagnoses and, for each diagnosis, a predicted probability that the diagnosis is accurate. That DAM assistant may also recommend additional parameters to monitor, diagnostic tests to perform, imaging studies to perform, and standard of care to be initiated/administered. The dynamic differential diagnosis list may be continuously updated as the DAM assistant is provided with data, such as data corresponding to clinical assessments, physiological parameters, diagnostic test results, and imaging studies.

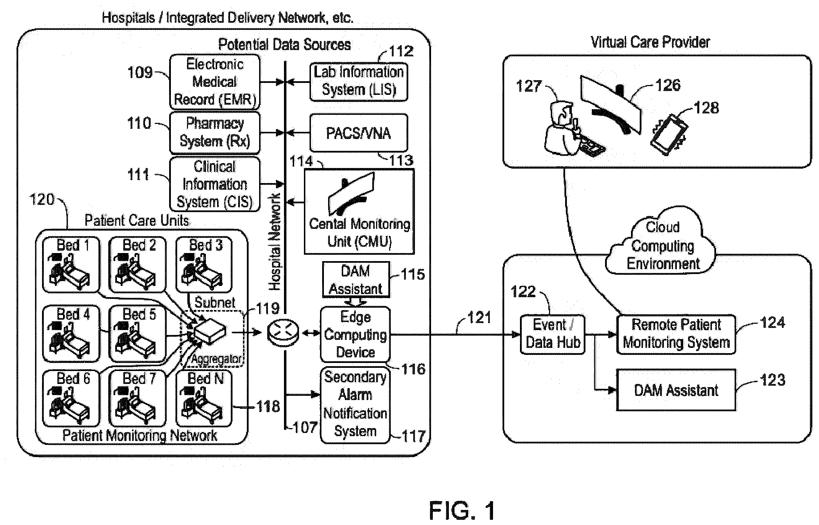

FIG. 1 is a diagram of an environment in which the DAM assistant operates and/or a care assistant operates, according to an embodiment. As shown in FIG. 1, the environment in which the DAM assistant and/or care assistant operates may include a hospital environment, a cloud computing environment, and a virtual care provider environment.

The hospital environment may include a hospital network 107 connected to a hospital Electronic Medical Record (EMR) database 109, a pharmacy system 110, a clinical information system 111, a lab information system 112, a Picture Archival System / Vendor Neutral Archive (PACS/VNA) 113, a central monitoring unit 114, an edge computing device 116, a secondary alarm notification system 117, and patient care units 120. The EMR database 109 may provide data corresponding to patients' demographics and medical history. The pharmacy system 110 may provide data corresponding to pharmaceutical associated with patients. The lab information system 112 may provide data indicating to diagnostic test results associated with patients. The PACS/VNA 113 may provide data associated with imaging studies associated with patients. The departmental clinical information system 111 may provide data indicating clinical observations and scores associated with patients. These systems may serve as data sources to a local edge computing device 116 which may include an HL7 integration engine.

Physiological patient monitors and other point of care devices such as ventilators and infusion pumps may be present in patient rooms 118 which are part of one or more patient care units 120 which in turn are connected to local data aggregator or monitoring gateway 119. According to an embodiment, the DAM assistant 115 may be deployed on the edge computing device 116 and may be notifying users of Central Monitoring Units 114 and nurses/physicians via a secondary alarm notification system 117. According to an embodiment, the DAM assistant 123 may be deployed on the cloud as a companion service to a remote patient monitoring system 124 and notifying clinical users 127 of a virtual care remote display 126 or mobile device 128. The remote patient monitoring system 124 could be many things such as a Virtual Care Solution. The hospital environment may include additional systems that integrate and display information from different device vendors, electronic health records, lab systems and treatment notes, etc.

The patient care units 120 may include one or more patient monitoring devices that monitor physiological parameters of a patient. Non-limiting embodiments of patient monitoring devices include a non-invasive blood pressure monitor, a blood oxygen monitor, an electrocardiogram, an electroencephalogram, a temperature monitor, a heart rate monitor, a respiration rate monitor, a carboxyhemoglobin monitor, an end-tidal carbon dioxide monitor, a heart rhythm monitor, a cardiac output monitor, invasive blood pressure monitor, and a heart rate variability monitor.

EMR database 109 may be an external database accessible by EMR module via a secured hospital interface, or EMR database 109 may be a local database (e.g., housed on a device of the hospital). EMR database 109 may be a database stored in a mass storage device configured to communicate with secure channels (e.g., HTTPS and TLS), and store data in encrypted form. Further, the EMR software is configured to control access to patient electronic medical records such that only authorized healthcare providers may edit and access the electronic medical records. An EMR for a patient may include patient demographic information, family medical history, past medical history, preexisting medical conditions, current medications, allergies, surgical history, past medical screenings and procedures, past hospitalizations and visits, etc.

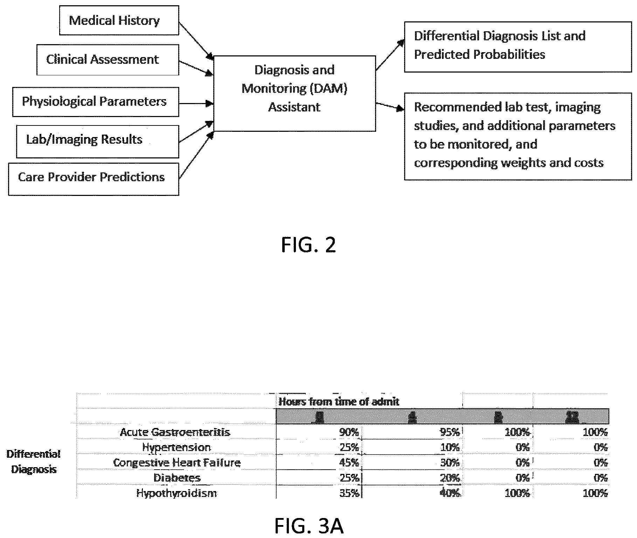

FIG. 2 is an input/output diagram of the DAM assistant, according to an embodiment. As shown in FIG. 2, the DAM assistant may input medical history, clinical assessment information, device data feeds, test results, and care provider predictions, among other forms of relevant data, for one or more patients. A patient's medical history may include previously diagnosed conditions, such as high blood pressure and/or obesity, current medications, and relevant family medical history. Clinical assessment may be based on observations made by a healthcare provider at admission. For example, the healthcare provider may observe that a patient appears swollen, disoriented, sweaty, etc. Device data feeds may include data feeds provided by medical devices monitoring physiological parameters of the one or more patients. For example, the device data feeds may include blood oxygen level (SP02) data and heart rate data for a patient. Lab/imaging results may include results of diagnostic labs, such as blood test, and imaging studies, such as the results of a computerized tomography. Care provider predictions may include a care provider's predicted probability that a diagnosis is accurate.

Using the above discussed inputs, the DAM assistant may generate a differential diagnosis list along with a predicted probability for each diagnosis on the list. The predicted probability may indicate a predicted probability that the diagnosis is accurate. The DAM assistant may utilize an artificial intelligence-based diagnosis (AID) model that is trained to generate the differential diagnosis list and the associated predicted probabilities using available training data sets. For example, training data may include medical history, clinical assessment information, device data feeds, and test result inputs that are labeled with differential diagnoses lists and predicted probabilities. The AID model may also be trained based on feedback from care providers using the DAM assistant, as discussed below.

Once a differential diagnosis list and associated predicted probabilities are generated by the DAM assistant, the DAM assistant may provide the healthcare provider with an opportunity to input revised predicted probabilities, based on the healthcare provider's expertise and understanding of the patient, to obtain aided probabilities (user predictions). The AID model may then be trained based on the aided probabilities, thus incorporating the care provider's feedback in future diagnoses. As such, future outputs of the AID model may be influenced by the inputs provided by the care provider, which may increase the accuracy of the AID model. The user may also choose to override the predicted probabilities with the aided probabilities and/or add additional diagnoses not shown in the DAM along with corresponding predicted probabilities. Overriding the predicted probabilities with the aided probabilities may include inputting the aided probabilities into the model and the model generating all associated information based on the newly input probabilities. When the predicted probabilities are overridden, the DAM assistant may continue to operate the AID model in parallel based on the originally generated predicted probabilities so a user may refer back to the unaided predictions, recommendations, and weights.

For one or more of the diagnoses on the differential diagnosis list, the DAM assistant may provide recommendations to increase the care provider's probability of an accurate diagnosis. The recommendations may include diagnostic test to be performed, imaging studies to be performed, additional physiological parameters to be monitored, and additional clinical assessments to be performed. The DAM assistant may also provide a weight for each of the recommendations. For example, a diagnosis of acute gastroenteritis may have a predicted probability of 45% without considering a heart rate of the patient, and the predicted probability may either increase to 55% (+10%) when the heart rate data indicates acute gastroenteritis or decrease to 40% (-5%) when the heart rate data does not indicate acute gastroenteritis. As such, the DAM assistant may determine that heart rate monitoring has a weight of 20 (based on 15% being added to 5%). As another example, the predicted probability of acute gastroenteritis may change from 45% to either 75% (+30%) or 25% (-20%) based on blood test results. That is, based on the blood test results indicating acute gastroenteritis, the predicted probability may increase to 75%, or based on the blood test results indicating that no acute gastroenteritis, the predicted probability may decrease to 25%. As such, the DAM assistant may determine that a blood test has a weight of 50 (based on 30% being added to 20%).

The DAM assistant may determine a cost to implement a recommendation, as well as a relative benefit to cost. For example, if test A cost $10 and has a weight of 10 and test B cost $10 and has a weight of 5, the benefit to cost for test A is 1 while the benefit to cost for test B is 0.5. This information may be provided to the care provider by the DAM assistant to assist in selecting an economical care plan.

The differential diagnosis list and corresponding predicted probabilities may be autonomously updated by the DAM assistant based on data being input into the DAM assistant. For example, the DAM assistance may output an initial differential diagnosis list, along with corresponding recommendations, based on initial/admission diagnosis data, clinical conditions data, and care provider observation data, because this may be the only available data at the time. Based on this initial output, a care provider may order a recommended diagnostic test. Once the results from the recommended diagnostic test are input into the DAM assistant, the DAM assistant may change the predicted probabilities of one or more diagnoses on the differential diagnosis list based on relevant information obtained from the test results. In response to the diagnostic test results, the DAM assistant may also adjust the recommended tests, studies, and additional parameters to be monitored, as well as their associated weights. According to an aspect of the disclosure, diagnoses may be added and/or removed from the differential diagnosis list based on the diagnostic test results. Results of an imaging study or information from a monitored parameter may also affect the differential diagnosis list, the corresponding predicted probabilities, and the recommendations, in a similar manner as discussed above with respect to the diagnostic test results.

In a healthcare environment, care providers may have an overwhelming amount of information to consider when caring for one or more patients. As such, it may be difficult for a care provider to retain, understand, and process this information in an appropriate manner to make informed decisions. This chaotic environment has the potential to cause errors that will decrease the quality of care for patient. The DAM assistant can be used to compile and process all of this information to provide a care provider with concise and useful information that can be easily retained, understood, and used to make informed decisions. Through using the DAM assistant, a care provider will be assisted in diagnosing a patient by the generated differential diagnosis list. The care provider will also be assisted in which parameters to monitor, diagnostic tests to perform, and imaging procedures to perform. As such, a quality of care may be improved by the DAM assistant through ensuring that the care provider is aware of all relevant diagnoses, as well as additional parameters to monitor, test to perform, and imaging studies to perform to assist in accurately diagnosing the patient. Further, the accuracy of the DAM assistant may increase over time by training the AID model of the assistant based on user inputs provided to the DAM assistant, thus further increasing quality of care for patients.

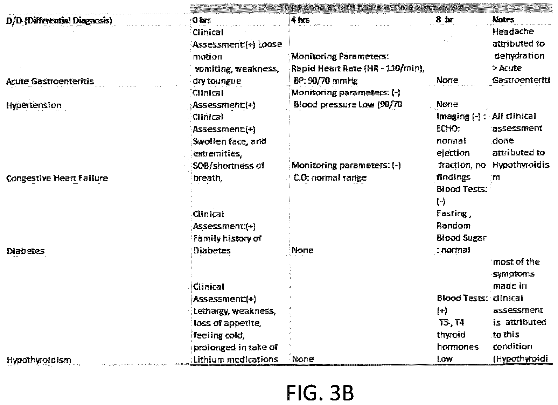

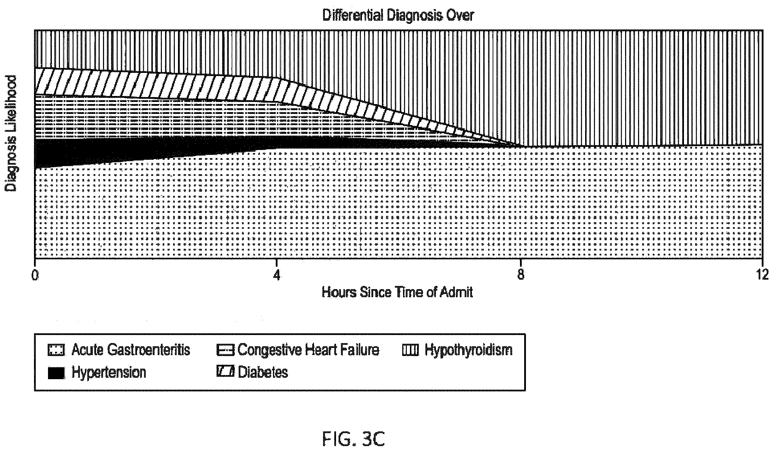

FIGs. 3A through 3C are directed to an example in which the DAM assistant assists a care provider in diagnosing a patient, according to an embodiment. FIG. 3A is a chart of a differential diagnosis list and corresponding predicted probabilities over a 12 hour period. FIG. 3B is a chart of the differential diagnosis list, along with parameters monitored and test performed over the 12 hour period. In FIG. 3B, (+) indicates that the information increased the predicted probability and (-) indicates that the information decreased the predicted probability. FIG. 3C is a graph of changes in the predicted probabilities of the diagnoses on the differential diagnosis list over the 12 hour period.

In the example of FIGs. 3A-3C, the patient is a 74 year old diabetic female that is wheeled into the ER and found to have the following signs and symptoms during the clinical assessment: disoriented, sweating, swollen face, dry tongue and extremities, shortness of breath, weakness and lethargy, loss of appetite, tremor, feeling cold, headache, and loose motion. The patient had a fever, body ache, and running nose 10 days prior for a period of 3 days. The patient is taking the following medication hydrochlorothiazide, lithium, and risperidone. The DAM assistant may input the existing condition (diabetes), medications taken (hydrochlorothiazide, lithium, and risperidone), age (74), and fever, body ache, and running nose 10 days prior for a period of 3 days as medical history information. This data may be imported from databases such as an electronic medical record (EMR) and/or input by a care provider. The DAM assistant may also input the disoriented, sweating, swollen face, dry tongue and extremities, shortness of breath, weakness and lethargy, loss of appetite, tremor, feeling cold, headache, and loose motion as clinical assessment information.

Based on the above inputs, which represent the data input into the DAM model at hour 0 (0 hours since admittance) as shown in FIGs. 3A-3C, the DAM assistant may generate the following candidate differential diagnosis list: acute gastroenteritis, hypertension, congestive heart failure, diabetes, and hypothyroidism. The DAM assistant may generate the differential diagnosis list using the AID model. The AID model may utilize a neural network, such as a convolutional neural network, trained using any of supervised, semi-supervised, unsupervised, and reinforcement learning techniques. The AID model may be trained to generate a differential diagnosis list and corresponding predicted probabilities based on inputs of medical history data, clinical assessment data, physiological parameter data, and/or test results data. For example, the AID model may be trained based on labeled datasets in which medical history data, clinical assessment data, physiological parameter data, and/or test results data is labeled with a differential diagnosis list including one or more diagnoses. The AID model may also be trained based on care provider predictions, such as when a care provider revises a predicted probability generated by the AID model.

According to an embodiment, the AID model may utilize multiple neural networks that are trained based on different training data. For example, a first neural network may be trained to generate the differential diagnosis list and corresponding predicted probabilities. A second neural network may be trained to recommend additional parameters to monitor and test to preform, along with associated weights. According to an embodiment, the AID model may include a neural network in which a first portion of layers are trained to generate the differential diagnosis list, and the first portion and a second portion of layers are trained to generate the differential diagnosis list and the predicted probabilities. According to an embodiment, the AID model may include a neural network in which a first portion of layers are trained to generate the recommended parameters, and the first portion and a second portion of layers are trained to generate the recommended parameters and the associated weights.

As shown in FIG. 3A, the DAM assistant generates a predicted probability that each diagnosis on the differential diagnosis is accurate. That is, the DAM assistant, by inputting the initial inputs into the AID model, may predict that the diagnosis of acute gastroenteritis is 90% accurate, hypertension is 25% accurate, congestive heart failure is 45% accurate, diabetes is 25% accurate, and hypothyroidism is 35% accurate based on the data input at hour zero. The total of all of the predicted probabilities may be greater than 100% due to comorbidities. That is, each predicted probability may be between 0% and 100% since the predicted probability is referring to the probability of the diagnosis to which it corresponds.

As shown in FIG. 3B, at hour 4 (4 hours since admittance), the physiological parameters of blood pressure, heart rate, and cardiac output (CO) are monitored. As also shown in FIG. 3B, at hour 4, new data indicates that the heart rate is high (110/min), the blood pressure is low (90/70), and the CO is normal. The DAM assistant may obtain this new data at hour 4 based on user inputs and/or by monitoring data streams of the physiological sensors. Using this physiological data, the AID model may update the predicted probabilities, as shown in FIG. 3A. That is, based on the rapid heart rate and low blood pressure, the DAM assistant determines that the predicted probability of acute gastroenteritis increases 5%; based on the low blood pressure data, the predicted probability of hypertension decreases 15% to 0%; and based on the normal CO, the predicted probability of congestive heart failure decreases 15%. Even though the new data input at hour 4 may not be directly related to the diabetes and hypothyroidism diagnoses, the AID model may adjust the predicted probabilities of these diagnoses, as shown in FIG. 3A, based on correlations learned through training as well as the changes in predicted probabilities of the other diagnoses.

At hour 8, as shown in FIG. 3B, echocardiogram imaging study results and blood test results are received and input into the AID model of the DAM assistant. The imaging study and blood test results may be input by a user or obtained from a database, such as a database in which the lab results are entered by a lab technician. Based on the echocardiogram study results indicating that the patient's heart is operating normally, the AID model changes the predicted probability of congestive heart failure to 0%, and based on the blood test results indicating normal sugar levels, the AID model changes the predicted probability of diabetes to 0%. Based on the predicted probability of hypertension, congestive heart failure, and diabetes being zero, and the other data (including the low hormone levels at hour 8) indicating that the patient is suffering from acute gastroenteritis and hypothyroidism, the AID model changes the predicted probability of acute gastroenteritis and hypothyroidism to 100%.

FIG. 3C is a visual representation of the predicted probabilities of each diagnosis over a period of 12h beginning at admittance of the patient. As shown in FIG. 3C, the predicted probabilities are refined to remove likely inaccurate diagnoses based on data that is input over time. New data being input at hours 4 and 8 is used for ease of explanation. The AID model of the DAM assistant may receive data at any interval. For example, the model may input data feeds from sensors monitoring physiological parameters of a patient and revise the predicted probabilities based on detecting abnormalities, or lack thereof, in the data feeds. Similarly, the AID model may revise the predicted probabilities in response to test results being input, without consideration of regular interval as used in the example.

According to an embodiment, the DAM assistant may recommend a standard of care to be initiated/administered. For example, the DAM assistant may recommend starting a sepsis protocol on the patient. According to another embodiment, the DAM assistant may generate and output a scoring system to assess patient deteriorating condition for a suspected sepsis.

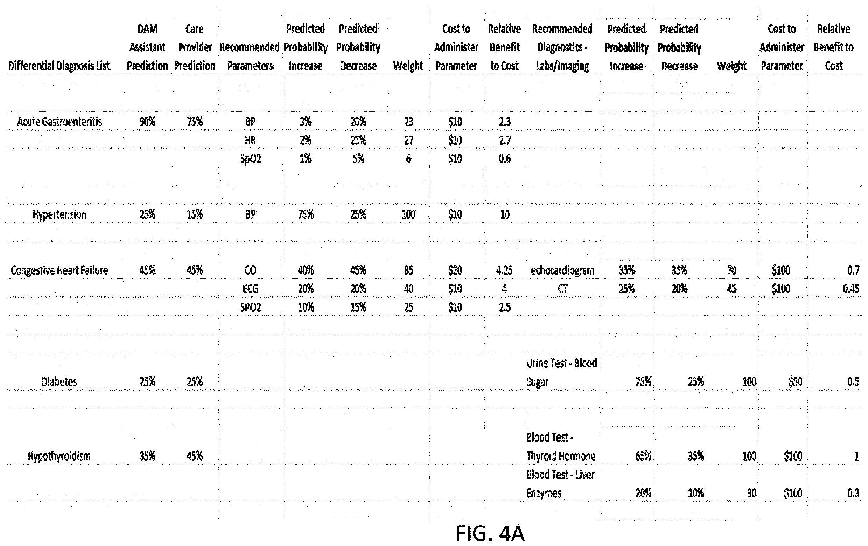

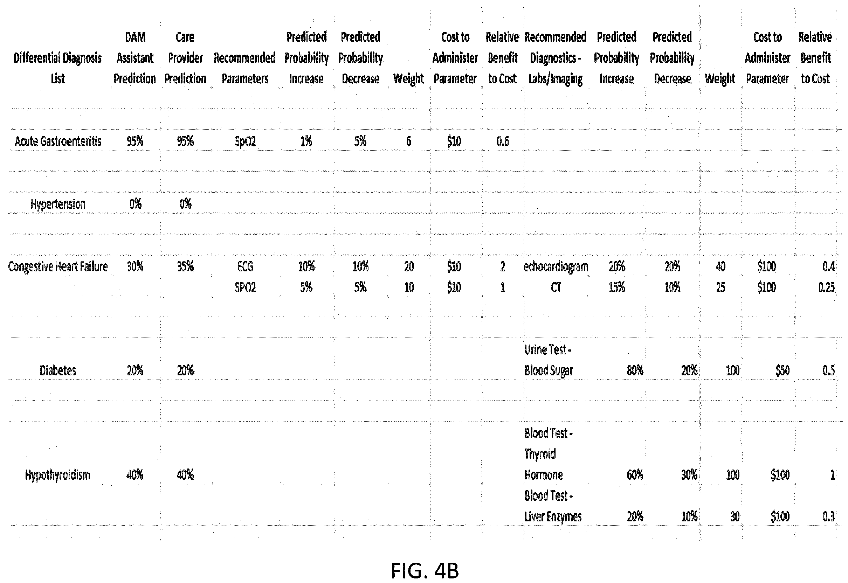

FIGs. 4A and 4B are tables of data produced by the DAM assistant at different points in time, according to an embodiment. The specific example shown in FIG. 4A corresponds to hour 0 of the example of FIGs. 3A though 3C. The specific example shown in FIG. 4B corresponds to hour 4 of the example of FIGs. 3A though 3C.

As shown in FIG. 4A, the DAM assistant may generate a differential diagnosis list and corresponding predicted probabilities for a patient. The DAM assistant may provide the differential diagnosis list and corresponding predicted probabilities to a care provider assigned to the patient. The DAM assistant may also receive and input care provider predicted probabilities. For example, as show in FIG. 4A, based on the care providers expertise and interaction with the patient, the care provider may predict that the probability of the patient suffering from acute gastroenteritis is 75%. As such, the care provider's prediction is 15% lower than the DAM assistant's prediction of 90%. According to an embodiment, the AID model of the DAM assistant may be trained using a difference between the DAM assistant's predicted probabilities and the care provider predicted probabilities.

As shown in FIG. 4A, the DAM assistant may recommend diagnostic test, imaging studies, and additional parameters for one or more of the diagnoses. Additionally, the DAM assistant may determine an estimated increase in the predicted probability of the diagnosis based on the recommendation indicating that the diagnosis is accurate, as well as an estimated decrease in the predicted probability of the diagnosis based on the recommendation indicating that the diagnosis is inaccurate. A weight may be assigned to each recommendation by adding the predicted increase and the predicted decrease. For example, as shown in FIG. 4A, monitoring the patient's ECG is predicted to either increase the predicted probability of congestive heart failure by 20% based on the ECG indicating that the diagnosis is positive and decrease the probability by 20% based on the ECG indicating that the diagnosis is negative. As such, ECG monitoring has a weight of 40 (20% + 20%). This weight is indicative of the estimated correlation between the recommendation and the diagnosis. For example, a patient with high blood pressure will be suffering from hypertension while a patient with normal or low blood pressure will not be suffering from hypertension. Accordingly, as shown in FIG. 4A, adding monitoring of blood pressure (BP) has a weight of 100 because this one recommendation is all that is needed to determine if the diagnosis is accurate or inaccurate. As another example, a liver enzyme test is not clearly indicative of hypothyroidism. As such, the liver enzyme blood test only has a weight of 30.

According to an embodiment, the predicted probability increases and the predicted probability decrease for the diagnostic tests, imaging studies, and additional parameters may be generated by the AID model, such as by a neural network of the AID model. For example, the model may be trained based on labeled training data including examples of scenarios in which results of diagnostic tests, imaging studies, and/or additional parameters received. The data may be labeled to indicate the predicted probability increase or the predicted probability decrease for one or more results received. The model may also be trained based on the care providers predictions. For example, once a result is input to the DAM model, a care provider may input a predicted probability that a diagnosis is accurate which is different than the predicted probability provided by the DAM model. The model can then be trained based on this difference when determining future predicted probability increases and decreases associated with diagnostic tests, imaging studies, and additional parameters.

The DAM assistant may also determine a relative benefit to cost to further assist a care provider in selecting a diagnostic test, imaging study, and/or additional parameter. As shown in FIG. 4A, each diagnostic test, imaging study, and/or additional parameter may be assigned a cost to administer. The cost information may be provided by a database that includes up to date hospital specific pricing. Using these costs and the weights, a relative benefit to cost can be determined by dividing the weight by the cost. For example, as shown in FIG. 4A, with respect to acute gastroenteritis, adding blood pressure monitoring has relative benefit to cost of 2.3 since monitoring blood pressure has a weight of 23 and a cost of $10.

According to an embodiment, the DAM assistant may also input additional lab test, imaging studies, and parameters that have not been recommended, as well as a corresponding predicted probability increase and decrease. For example, when a test in which the care provider believes would assist in diagnosing the patient is not recommended by the DAM assistant, the care provider may input the additional test and the associated predicted probability increase and decrease. The added information may then be displayed to the caregiver throughout the diagnosis process. The added information may also be used to train the AID model for future similar scenarios.

As discussed above, in the example of FIGs. 3A through 3C, the predicted probabilities of the diagnoses on the differential diagnosis list change at hour 4 in response to cardiac output, blood pressure, and heart rate being monitored. FIG. 4B is an example of data provided by the DAM assistant at hour 4 in response to the new cardiac output, blood pressure, and heart rate data being input into the DAM assistant.

As shown in FIG. 4B, since cardiac output, blood pressure, and heart rate are being monitored, monitoring these parameters are no longer recommended. Additionally, the increased/decreased probabilities associated with the recommendations are different than at hour zero due to new data being considered. For example, with respect to congestive heart failure, once the cardiac output has been considered, ECG data is less likely to affect the predicted probability. As such, the predicted probability increase and predicted probability decrease for adding ECG monitoring has changed from 20% and 20% to 10% and 10%, respectively.

According to an aspect of the disclosure, the DAM assistant may also recommend parameters to be removed from monitoring. To determine these parameters, the DAM assistant may track parameters that are being monitored and determine a value of the parameters. Based on the value being below a threshold, the DAM assistant may recommend ending monitoring of the parameter to prevent waste and unnecessary expenses. For example, if the DAM assistant determines that the SPO2 data has minimal value in assisting with the diagnosis, the DAM assistant may recommend ending monitoring of SP02.

According to an embodiment, the AID model may determine a value and a cost of parameters being monitored. Based on this information, the DAM assistant may determine a relative value to cost for each parameter being monitored. The relative value to cost may be provided to a care provider to assist in determine which parameters to end monitoring.

According to an embodiment, the final result of the diagnosis may be used for training the model. For example, with regard to the example shown in FIGs. 3A-3C, the DAM assistant may determine that the final diagnosis is accurate gastroenteritis and hypothyroidism. The DAM assistant may ask the user to confirm that the determined final diagnosis is accurate to ensure that training data is correct. The final diagnosis information may then be used to train the AID model for similar scenarios.

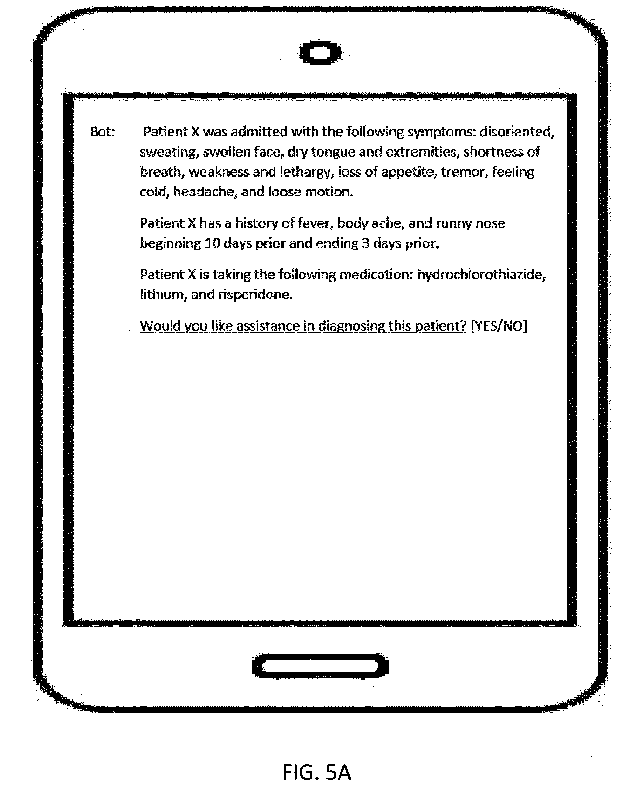

FIGS. 5A-5E show a chatbot interface of the DAM assistant, according to an aspect of the disclosure. According to a non-limiting embodiment, the DAM assistant may interact with a user via a computing device having a user or human-machine interface, such as a personal computer, a tablet computer, or a handheld device such as a cell phone. The user may receive data from the computing device and also input data into the computing device. FIGs. 5A-5E show different outputs of a chatbot provide on the computing device for interacting with the user.

FIG. 5A shows an initial message provided to a care provider (user) that is receiving the patient. The initial message may provide the user with relevant information for appropriately diagnosing and monitoring the patient, such as information corresponding to the clinical assessment and patient's medical history. The chatbot may also ask if the user would like assistance in diagnosing the patient. For example, as shown in FIG. 5A, the chatbot may state the following: "Patient X was admitted with the following symptoms: disoriented, sweating, swollen face, dry tongue and extremities, shortness of breath, weakness and lethargy, loss of appetite, tremor, feeling cold, headache, and loose motion. Patient X has a history of fever, body ache, and runny nose beginning 10 days prior and ending 3 days prior. Patient X is taking the following medication: hydrochlorothiazide, lithium, and risperidone. Would you like assistance in diagnosing this patient? [YES/NO]"

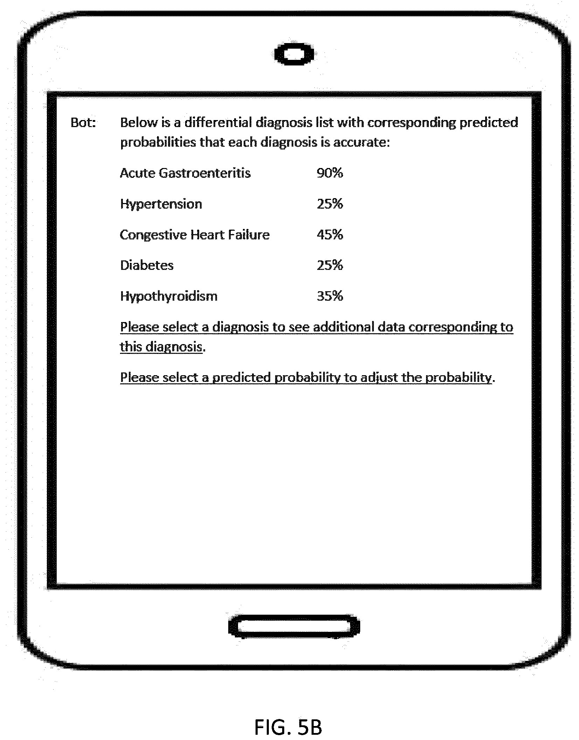

In response to the user selecting "yes" for assistance in diagnosing, the chatbot may output a differential diagnosis list and corresponding predicted probabilities that are generated by the DAM assistant, as shown in FIG. 5B. The user may select one or more of the predicted probabilities to input a care provider prediction that differs from the generated predicted probability. The user may also select a diagnosis to see additional information corresponding to the diagnosis.

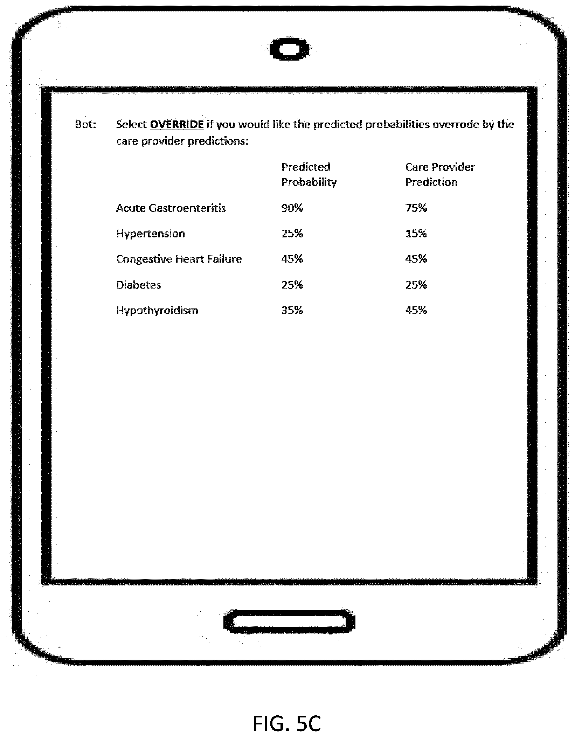

For example, in response to entering one or more care provider predictions, the chatbot may ask the user if they would like to override the predicted probabilities with the care provider predictions, as shown in FIG. 5C. If the user does not select override, the chatbot may display the generated predicted probabilities along with the care provider predictions during the diagnosis process. If the user selects override, the care provider predictions may be input into the AID model and new recommendations and weights may be generated based on the newly input probabilities. When the predicted probabilities are overridden, the AID model may also continue to generate data based on the initial prediction probabilities so this data can continue to be accessed by a user.

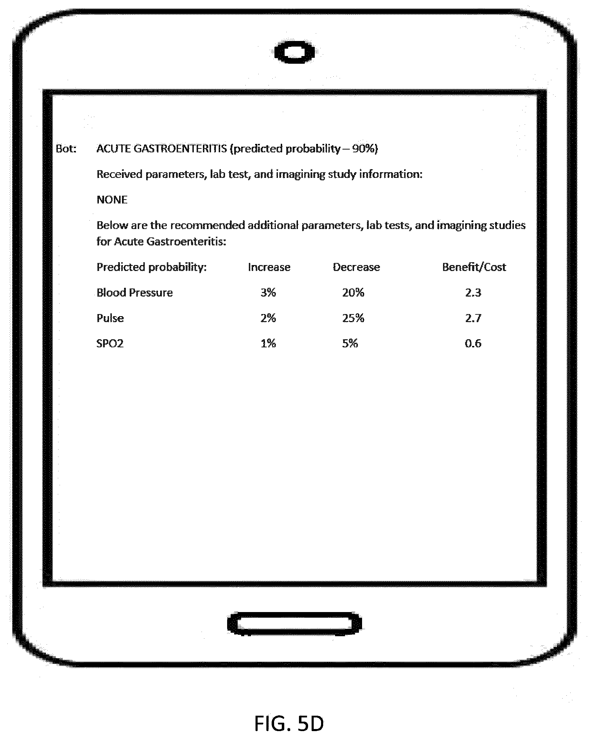

For example, in response to a user selecting acute gastroenteritis in FIG. 5B, the chatbot may output received parameters, diagnostic tests, and imaging study information, along with recommended parameters, diagnostic tests, and imaging study information, as shown in FIG. 5D. The example of FIG. 5D corresponds to hour 0 of FIGs. 3 and 4. As shown in FIG. 5D, since at hour 0 there are no parameters being monitored and no diagnostic test/imaging study results have been received, no information is displayed as having been received. Since the DAM assistant has recommended additional parameters to monitor, as shown in FIG. 4A, the chatbot recommends monitoring blood pressure, heart rate, and SP02. The chatbot may also provide the user with a predicted increase and decrease in predicted probability that the diagnosis is accurate, as well as relative benefit to cost, for each recommendation to assist the user in selecting the parameters.

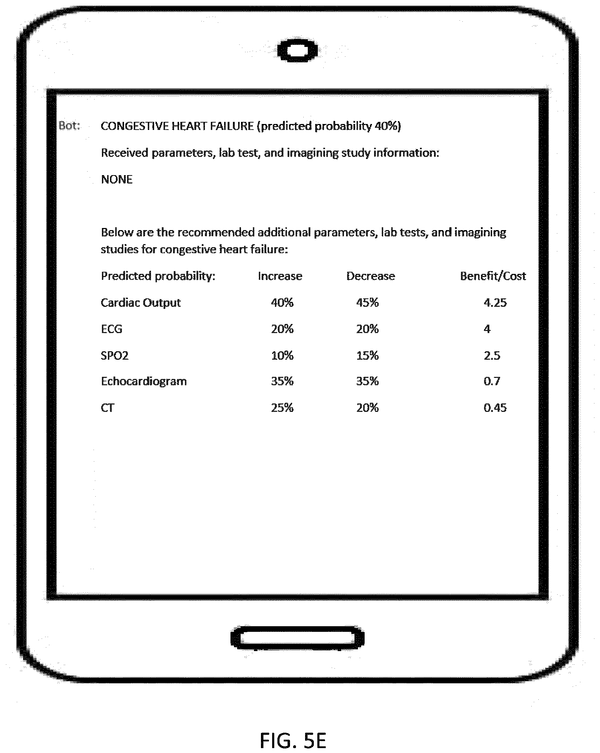

FIG. 5E shows an output of the chatbot in response to the use selecting congestive heart failure in FIG. 5B. As shown in FIG. 5D, the chatbot provides the user with recommendations of additional parameters to monitor and imaging studies to perform, along with corresponding predicted increases/decreases in the predicted probability as well as relative benefit to cost. By having this information readily available, the user is able to make a quick and informed decision, thus improving quality of care for the patient.

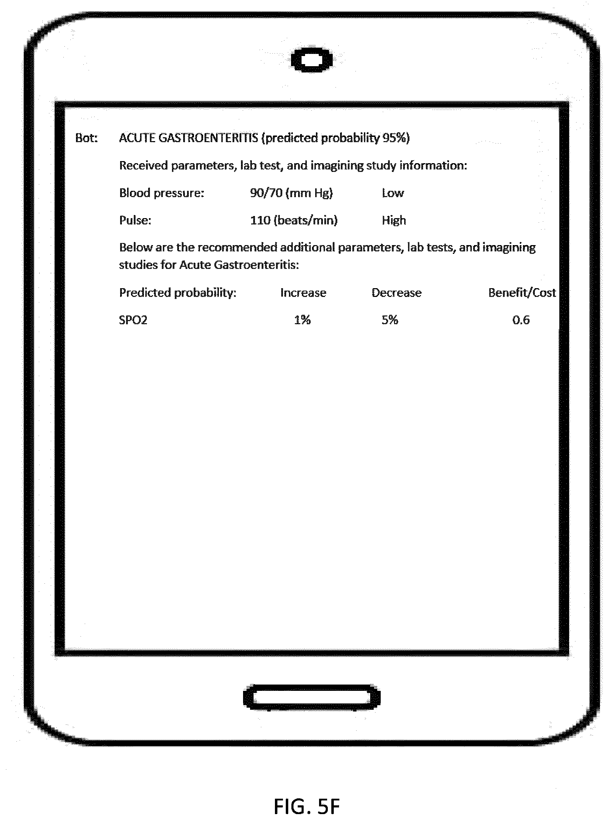

FIG. 5F shows an output of the chatbot in response to the user selecting acute gastroenteritis at hour 4 of the example shown in FIGs. 3 and 4. As shown in FIG. 5F, since blood pressure and heart rate are being monitored, the chatbot may output data corresponding to these parameters. The data may be displayed in real time or near real time and may be continuously updated based on a live data feed from the sensors received by the DAM assistant.

According to an embodiment, the DAM assistant may also display risk score data for the patient. For example, the risk score may be obtained from the EMR database and may be displayed by the chat bot interface to assist the user in diagnosing the patient.

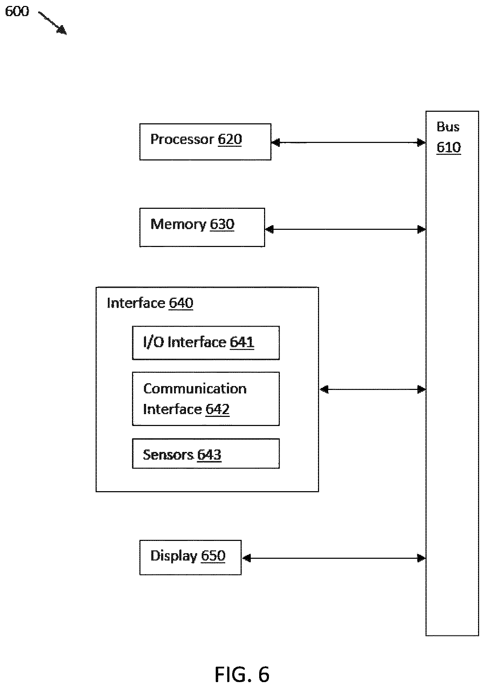

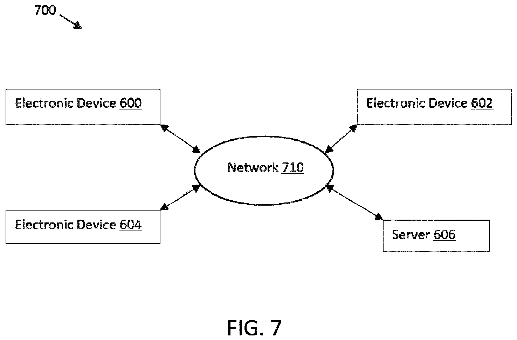

The DAM assistant may be deployed, partially deployed, operated, or accessed on electronic device 600 of Fig. 6 in a network environment 700 as shown in Fig. 7, according to an embodiment. Figs 6 and 7 are for illustration only, and other embodiments of the electronic device and environment could be used without departing from the scope of this disclosure.

As shown in FIG. 6 electronic device 600 may include at least one of a bus 610, a processor 620 (or a plurality of processors), a memory 630, an interface 640, and a display 650.

Bus 610 may include a circuit for connecting the components 620, 630, 640, and 650 with one another. Bus 610 may function as a communication system for transferring data between the components, or between electronic devices.

Processor 620 may include one or more of a central processing unit (CPU), a graphics processor unit (GPU), an accelerated processing unit (APU), many integrated core (MIC), a field-programmable gate array (FPGA), or a digital signal processing (DSP). Processor 620 may control at least one of other components of electronic device 600, and/or perform an operation or data processing relating to communication. Processor 620 may execute one or more programs stored in memory 630.

Memory 630 may include a volatile and/or a non-volatile memory. Memory 630 may store information, such as one or more commands, data, programs (one or more instructions), or applications, etc., that is related to at least one other component of the electronic device 600 and for driving and controlling electronic device 600. For example, commands or data may formulate an operating system (OS). Information stored in memory 630 may be executed by processor 620.

The application may include one or more embodiments as discussed above. These functions can be performed by a single application or by multiple applications that each carry out one or more of these functions.

Display 650 may include, for example, a liquid crystal display (LCD), a light emitting diode (LED) display, an organic light emitting diode (OLED) display, a quantum-dot light emitting diode (QLED) display, a microelectromechanical systems (MEMS) display, or an electronic paper display. Display 650 can also be a depth-aware display, such as a multi-focal display. Display 650 is able to present, for example, various contents (such as text, images, videos, icons, or symbols).

Interface 640 may include input/output (I/O) interface 641, communication interface 642, and/or one or more sensors 643. I/O interface 641 serves as an interface that can, for example, transfer commands or data between a user or other external devices and other component(s) of electronic device 600. The I/O interface 641 may provide a human-machine user interface which provides information to a user and receives information from the user.

Sensor(s) 643 may meter a physical quantity or detect an activation state of electronic device 600 and may convert metered or detected information into an electrical signal. For example, sensor(s) 643 may include one or more cameras or other imaging sensors for capturing images of scenes. The sensor(s) 643 may also include a microphone, a keyboard, a mouse, a touchscreen, one or more buttons for touch input, a gyroscope or gyro sensor, an air pressure sensor, a magnetic sensor or magnetometer, an acceleration sensor or accelerometer, a grip sensor, a proximity sensor, a color sensor (such as a red green blue (RGB) sensor), a bio-physical sensor, a temperature sensor, a humidity sensor, an illumination sensor, an ultraviolet (UV) sensor, an electromyography (EMG) sensor, an electroencephalogram (EEG) sensor, an electrocardiogram (EGG) sensor, an infrared (IR) sensor, an ultrasound sensor, an iris sensor, or a fingerprint sensor. The sensor(s) 643 can further include an inertial measurement unit. In addition, sensor(s) 643 can include a control circuit for controlling at least one of the sensors included here. Any of these sensor(s) 643 can be located within or coupled to electronic device 600. Sensor(s) 643 may be used to detect touch input, gesture input, hovering input using an electronic pen or a body portion of a user, etc.

Communication interface 642, for example, may be able to set up communication between electronic device 600 and an external electronic device (such as a first electronic device 602, a second electronic device 604, or a server 606 as shown in Fig. 7). As shown in Fig. 7, communication interface 642 may be connected with a network 710 through wireless or wired communication architecture to communicate with an external electronic device. Communication interface 642 may be a wired or wireless transceiver or any other component for transmitting and receiving signals.

Fig. 7 shows an example network configuration 700 according to an embodiment. Electronic device 600 of Fig. 6 may be connected with a first external electronic device 602, a second external electronic device 604, or a server 606 through network 710. Electronic device 600 may be wearable device, an electronic device-mountable wearable device (such as an FIMD), etc. When electronic device 600 is mounted in the electronic device 602 (such as the FIMD), electronic device 600 may communicate with electronic device 602 through communication interface 642. Electronic device 600 may be directly connected with electronic device 602 to communicate with electronic device 602 without involving a separate network. Electronic device 600 may also be an augmented reality wearable device, such as eyeglasses, that include one or more cameras.

The first and second external electronic devices 602 and 604 and server 506 may each be a device of a same or a different type than electronic device 600. According to some embodiments, server 606 may include a group of one or more servers. Also, according to some embodiments, all or some of the operations executed on electronic device 600 may be executed on another or multiple other electronic devices (such as electronic devices 602 and 604 or server 606). Further, according to some embodiments, when electronic device 600 should perform some function or service automatically or at a request, electronic device 600, instead of executing the function or service on its own or additionally, can request another device (such as electronic devices 602 and 604 or server 506) to perform at least some functions associated therewith. The other electronic device (such as electronic devices 602 and 604 or server 606) may be able to execute the requested functions or additional functions and transfer a result of the execution to electronic device 600. Electronic device 600 can provide a requested function or service by processing the received result as it is or additionally. To that end, a cloud computing, distributed computing, or client-server computing technique may be used, for example. While Figs 6 and 7 show that electronic device 600 including communication interface 642 to communicate with external electronic devices 602 and 604 or server 606 via the network 710, electronic device 600 may be independently operated without a separate communication function according to some embodiments.

Server 606 may include the same or similar components 610, 620, 630, 640, and 650 as electronic device 600 (or a suitable subset thereof). Server 506 may support driving electronic device 600 by performing at least one of operations (or functions) implemented on electronic device 600. For example, server 606 can include a processing module or processor that may support processor 620 of electronic device 600.

The wireless communication may be able to use at least one of, for example, long term evolution (LTE), long term evolution-advanced (LTE-A), 5th generation wireless system (5G), millimeter-wave or 60 GFIz wireless communication, Wireless USB, code division multiple access (CDMA), wideband code division multiple access (WCDMA), universal mobile telecommunication system (UMTS), wireless broadband (WiBro), or global system for mobile communication (GSM), as a cellular communication protocol. The wired connection may include, for example, at least one of a universal serial bus (USB), high definition multimedia interface (HDMI), recommended standard 232 (RS-232), or plain old telephone service (POTS). The network 710 includes at least one communication network, such as a computer network (like a local area network (LAN) or wide area network (WAN)), Internet, or a telephone network.

Although Fig. 7 shows one example of a network configuration 700 including an electronic device 600, two external electronic devices 602 and 604, and a server 606, various changes may be made to Fig. 7. For example, the network configuration 700 could include any number of each component in any suitable arrangement. In general, computing and communication systems come in a wide variety of configurations, and Fig. 7 does not limit the scope of this disclosure to any particular configuration. Also, while Fig. 7 shows one operational environment in which various features disclosed in this patent document can be used, these features could be used in any other suitable system.

The event detection method may be written as computer-executable programs or instructions that may be stored in a medium.

The medium may continuously store the computer-executable programs or instructions, or temporarily store the computer-executable programs or instructions for execution or downloading. Also, the medium may be any one of various recording media or storage media in which a single piece or plurality of pieces of hardware are combined, and the medium is not limited to a medium directly connected to electronic device 600, but may be distributed on a network. Examples of the medium include magnetic media, such as a hard disk, a floppy disk, and a magnetic tape, optical recording media, such as CD-ROM and DVD, magneto-optical media such as a floptical disk, and ROM, RAM, and a flash memory, which are configured to store program instructions. Other examples of the medium include recording media and storage media managed by application stores distributing applications or by websites, servers, and the like supplying or distributing other various types of software.

The DAM assistant may be provided in a form of downloadable software. A computer program product may include a product (for example, a downloadable application) in a form of a software program electronically distributed through a manufacturer or an electronic market. For electronic distribution, at least a part of the software program may be stored in a storage medium or may be temporarily generated. In this case, the storage medium may be a server or a storage medium of server 606.

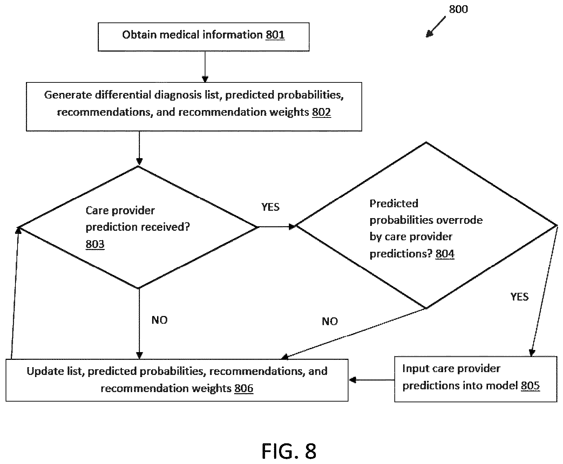

FIG. 8 is a flowchart of a method 800 of diagnosing and monitoring a patient, according to an embodiment. The method may be implemented on electronic device 600, in network environment 700, and/or in the environment of FIG. 1. The method 800 may be implemented using the DAM assistant.

At operation 801, medical information may be obtained. Medical information may include a clinical assessment based on observations made by a healthcare provider when the patient is admitted. The medical information may also include a patient's medical history and prescription drug usage. For example, a patient's medical history may include previously diagnosed conditions, such as high blood pressure and/or obesity, current medications, and relevant family medical history; and the clinical assessment observation may include that patient appears swollen, disoriented, and sweaty.

At operation 802, a differential diagnosis list may be generated based on the medical information obtained in operation 801. The differential diagnosis list may be generated by a model such as an AI model, a rules-based model, or a combination of the two. The model may input the medical information and output the differential diagnosis list. An example differential diagnosis list may include acute gastroenteritis, hypertension, congestive heart failure, diabetes, and hypothyroidism.

At operation 802, predicted probabilities may be generated by the model for each diagnosis on the differential diagnosis list. The predicted probabilities may indicate an estimated accuracy of a corresponding diagnosis. For example, the model may predict that, based on the input medical information input at operation 801, there is a 90% chance that the patient is suffering from acute gastroenteritis.

At operation 802, recommendations for one or more of the diagnoses may be generated. The recommendations may include additional parameters to monitor, diagnostic test to perform, and imaging studies to perform. For example, based on the medical information input at operation 801, the model may recommend monitoring blood pressure, heart rate, and blood oxygen levels to further confirm the diagnosis of acute gastroenteritis.

At operation 802, weights may be generated for each of the recommendations. A recommendation weight may indicate a change in a predicted probability of a diagnosis based on the information corresponding to the recommendation being considered in the diagnosis. A recommendation weight may be determined by determining a predicted probability increase based on the recommendation indicating that the diagnosis is accurate and a predicted probability decrease based on the recommendation indicating that the diagnosis is inaccurate. The predicted probability increase and the predicted probability decrease may be combined to determined the weight of the recommendation.

A benefit to cost may also be generated for each recommendation. The benefit to cost may be determined by dividing a weight of the recommendation by the cost to implement the recommendation. For example, if a first recommendation costs $100 and has a weight of 5, the benefit to cost would be 0.05, and if a second recommendation cost $10 and has a weight of 20, the benefit to cost would be 2. A care provider can then use this information in determining an economical diagnosis plan.

At operation 803, once a care provider is provided with the differential diagnosis list and corresponding predicted probabilities, the care provider may optionally input care provider predictions. The care provider predictions may be input when a care provider believes that a predicted probability generated by the model is inaccurate. For example, a care provider may believe that a 90% predicted probability of acute gastroenteritis is high, and therefore may input a care provider prediction of 50%. This prediction may be used to train the model for generating future predicted probabilities. This care provider prediction may also be used to adjust the current predicted probabilities.

The care provider may also input one or more additional diagnosis that is not listed on the generated differential diagnosis list, along with a predicted probability for the diagnosis. These added diagnoses may then be added to the diagnosis list that is continuously updated in operation 804. The addition of the diagnoses may also be used to train the AID model for future predictions.

At operation 804, if care provider predictions have been input, a user may select to override the predicted probabilities with the care provider predictions. For example, the chatbot may ask the user if he/she would like to override the predicted probabilities with the care provider predictions. The override by the care provider may be documented along with a rational for why. This information may be used to train the AID model.

At operation 805, based on the user selecting to override the predicted probabilities with the care provider predictions, the care provider predictions may be entered into the AID model as new predicted probabilities, which may then generate new recommendations and weights based on the newly input probabilities. For example, the model may input the care provider prediction of 50% and adjust the predicted probability of acute gastroenteritis to 50%. Since the predicted probability of acute gastroenteritis is now much lower, the model may determine the predicted probability of other diagnoses are higher. Additionally, since the predicted probability of acute gastroenteritis is decreased 30%, the recommendations will likely have a larger effect on the predicted probability. Accordingly, the model may also adjust the weights of the recommendations in response to the care provider prediction.

At operation 806, once the diagnosis list is set, the model may continuously update the differential diagnosis list, the predicted probabilities, the recommendations, and/or the recommendation weights. According to an embodiment, the model may input real time or near real time data feeds of parameters being monitored. The model may analyze this data to determine whether it indicates that the differential diagnosis list, a predicted probability, a recommendation, and/or a recommendation weight should be adjusted. In response to a determination of an adjustment, the item may be autonomously updated without any user intervention. For example, blood pressure may be monitored in response to a recommendation of blood pressure monitoring. In response to the monitored blood pressure being low, the model may decrease the predicted probability of hypertension. According to another embodiment, a user may monitor a parameter and input information into the model based on the monitoring, such as that the blood pressure is low. The model may then use this information to adjust the predicted probabilities, and notify the care team when applicable.

The model may also be continuously updated based on diagnostic test and imaging study results. For example, once a diagnostic test is performed, a diagnostic test result may be input into a diagnostic information system. The diagnostic information system may then provide this information into the model. For example, a blood test may indicate that a patient has high thyroid hormones. Based on this information being input into the model, the model may update the differential diagnosis list and/or predicted probability of hypothyroidism to be lower. Similarly, once an imaging study is performed, the results may be input into a program or database that provides the results to the model. The model may then update the differential diagnosis list and/or predicted probabilities in response to receiving the results. For example, whenever the DAM assistant notices a change in the patient, the assistant may notify the provider and care team of changes (e.g. diagnosis, parameter recommendations). The notification may be configurable based on thresholds.

According to an embodiment, a list of recommended parameters in which monitoring should be ended may also be continuously generated and updated as the DAM assistant is receiving new data. For example, based on the DAM assistant determine that monitoring ECG has minimal or no value in diagnosing acute gastroenteritis, the DAM assistant may recommend ending ECG monitoring. The DAM assistant may determine value of a monitored parameter using the AID model. For example, an amount in which the AID model uses the ECG data to contribute the to diagnosis may be determined. This amount may then be compared to a predetermined threshold or a cost of the monitoring.

The care provider may optionally input care provider predictions in response to the updated list. The care provider predicted may be used by the model as discussed above with respect to operation 803.

According to embodiments, operations 804 and 805 may be repeated until the patient is fully diagnosed. According to another embodiment, the DAM assistant, using above discussed model or a separate model, may continue to assist care provider in monitoring the patient once the diagnosis is obtained. For example, the DAM assistant may continue to monitor the patient's physiological parameters and/or recommend a monitoring plan. For example, once the patient is diagnosed with the final diagnosis, the DAM assistant may recommend monitoring intervals for parameters being monitored. The DAM assistant may also track monitoring time to ensure they are within the recommended intervals. Any changes in te patient while being surveilled by the system may result in a new diagnosis or care plan recommendations.

According to an embodiment, the DAM assistant may continue to assist a care provider in monitoring the patient once the patient has been discharged. The DAM assistant may recommend a post discharge monitoring plan including physiological parameters to monitor as well as monitoring intervals. The DAM assistant may input data from the at home monitoring equipment to further assist in monitoring the patient. For example, the DAM assistant may track monitoring intervals as well as abnormal monitoring data. The DAM assistant may also provide recommendations based on the abnormal monitoring data.

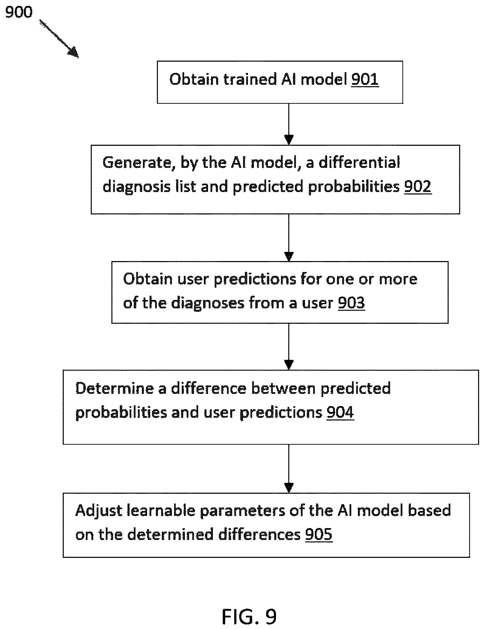

FIG. 9 is a flow chart of a process 900 for training an AI model to generate a differential diagnosis list and corresponding predicted probabilities, according to an embodiment. The process 900 of FIG. 9 may be used to train a model of the DAM assistant, such as the AID model, a neural network of the AID model, or a portion of a neural network of the AID model.

At operation 901, a trained AI model is obtained. The AI model may be trained to generate a differential diagnosis list and corresponding predicted probabilities based on inputs of medical history, clinical assessment, physiological parameters, diagnostic test results, and/or imaging study results. The AI model may be trained based on labeled datasets in which medical history data, clinical assessment data, physiological parameter data, and/or test results data is labeled with a differential diagnosis list including one or more diagnoses. The AI model may utilize a neural network, such as a convolutional neural network, trained using any of supervised, semi-supervised, unsupervised, and reinforcement learning techniques

At operation 902, the AI model may generate a differential diagnosis list and corresponding predicted probabilities based on inputs of medical history, clinical assessment, physiological parameters, diagnostic test results, and/or imaging study results. For example, the AI model may generate the differential diagnosis list described in hour 0 of FIGs. 3A-3C based on medical history information and clinical assessment information.

At operation 903, a user may input predicted probabilities that the diagnoses on the differential diagnosis list are accurate. For example, the user may input a user prediction for one or more of the diagnoses based on the user's understanding of the patient's condition.

At operation 904, a different between the predicted probabilities and the user prediction may be determined. For example, if the AI model predicts that the probability of the patient having acute gastroenteritis is 90% and the user prediction for acute gastroenteritis is 80%, the difference would be 10%.

At operation 905, the learnable parameters of the AI model may be adjusted based on the determined difference. For example, the learnable parameters of the AI model may be adjusted using known training techniques in which the user prediction is used a ground truth.

The training process 500 may be repeated indefinitely as more user prediction are obtained through use of the DAM assistant to further increase the accuracy of the AI model used therein.

According to an aspect of the disclosure, a patient care assistant may be implemented once a diagnosis is determined to assist a care team in monitoring and treating a diagnosed patient. The patient care assistant may be integrated with the DAM assistant or implemented as a standalone assistant.

Once a patient is diagnosed, dynamically controlling care of the patient may increase the quality of patient care while also decreasing waste (e.g., unnecessary use of medical devices, unnecessary tests, unnecessary costs, unnecessary time spent at facility). Dynamically controlling care of the patient may include reassessing and/or adjusting a care plan based on receiving new information corresponding to the patient (e.g., test results, monitoring data).

According to an aspect, a patient care model may generate a care plan for a patient based on patient information available at the time of diagnosis. For example, the patient care model may input a patient's diagnosis, medical history, clinical assessment information, device data feeds, test results (e.g., lab tests, imaging tests, etc.), among other forms of relevant data, and, based on this input data, the patent care model may generate and output an initial care plan.

According to another aspect, the patient care assistant may input an initial care plan. According to an aspect, the initial care plan may be a standardized care plan obtained from a database. For example, a hospital may have standardized care plans that can be selected based on a diagnosis as well as additional information corresponding to a patient. That is, a standardized care plan may be provided for a patient diagnosed with bronchitis who is over 55 years old, while a separate standardized care plan may be provided for a patient diagnosed with bronchitis who is under 55 years old. According to another aspect, the initial care plan may be created by a member of the care team. For example, a physician overseeing the patient may provide an initial care plan based on the diagnosis as well as their knowledge of the patient. According to another aspect, a standardized care plan may be provided to a care team member and the care team member may revise the standardized care plan to obtain the initial care plan that is input into the patient care model.

The initial care plan may be input into the care assistant to provide a starting point for care of the patient. The initial care plan may be distributed to members of a care team so consistent care is provided to the patient from all the members of the care team.

Upon receiving new information corresponding to the patient, the care assistant may adjust the care plan based on the new information. For example, responsive to receiving new information indicating that a patient parameter has stabilized, the care assistant may remove or suggest to remove additional monitoring of the stabilized parameter from the treatment plan. According to another example, responsive to receiving new information indicating that a patient parameter has stabilized, the care assistant may remove or suggest to remove future tests related to the patient parameter from the treatment plan. According to another example, responsive to receiving new information indicating that a patient parameter is outside of a preset range, the care assistant may add or suggest to add additional monitoring or tests to the treatment plan. By continuously adjusting or suggesting to adjust the care plan based on new information, human errors, which may results in best practices not being followed, may be avoided.

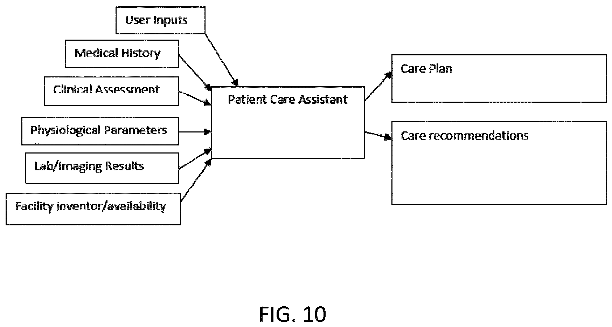

FIG. 10 is an input/output diagram of the patient care assistant, according to an aspect. As shown in FIG. 10, the care assistant may input medical history, clinical assessment information, device data feeds, test results, under inputs, and facility inventory/availability, among other forms of relevant data, for one or more patients. A patient's medical history may include previously diagnosed conditions, such as high blood pressure and/or obesity, current medications, and relevant family medical history. Clinical assessment may be based on observations made by a healthcare provider. For example, the healthcare provider may observe that a patient appears swollen, disoriented, sweaty, etc. Device data feeds may include data feeds provided by medical devices monitoring physiological parameters of the one or more patients. For example, the device data feeds may include blood oxygen level (SP02) data and heart rate data for a patient, among other parameters. Lab/imaging results may include results of diagnostic labs, such as blood test, and imaging studies, such as the results of a computerized tomography. User input may include additions, revisions, and removals of care recommendations.

Decision making of the care assistant may be provided by a patient care model. The patient care model may included AI based models as well a non-AI based models (e.g., statistical models, rules based algorithms). According to an aspect, the patient care model may utilize multiple sub-models.

A monitoring sub-model of the patient care model may include one or more AI models, such as neural networks, trained to recommend a patient monitoring plan based on a patient's diagnosis, medical history, clinical assessment information, device data feeds, test results, imaging results, outputs of the patient care model, among other forms of relevant data, for one or more patients. At an outset of treatment, the monitoring sub-model may input an initial patient monitoring plan of the initial care plan along with any patient information (e.g., diagnosis, parameter data, test results, etc.) to obtain a monitoring recommendation. The monitoring recommendation may include parameters to monitor, a timeframe/interval for monitoring the parameters, tests to be performed, timing of the tests, imaging to be performed, unit to be placed, among other known patient monitoring recommendations.

According to an aspect, a neural network of the monitoring sub-model may be trained to generate recommendations using supervised learning. The supervised learning process may use annotated training data in which patient information is annotated with a patient monitoring plan information. That is, training data may include a patient's diagnosis, medical history, clinical assessment information, device data feeds, test results, among other forms of relevant patient data. The ground truth may be obtained from the annotation of patient monitoring plan information. Based on the patient information inputs and the corresponding patient monitoring plan information ground truth, the neural network may be trained to generate recommended patient monitoring plans. For example, large datasets of real-world patient information and corresponding patient monitoring plans may be used to train a neural network of the monitoring sub-model. According to an aspect, the sub-model may also be trained based on use feedback. For example, the monitoring sub-model may be trained based on information corresponding to whether a care team member accepts, revises, or discards the recommendation or a portion thereof.

A treatment sub-model may include one or more AI models, such as neural networks, trained to recommend a treatment plan based on a patient's diagnosis, medical history, clinical assessment information, device data feeds, test results, and output of the patient care model, among other forms of relevant data, for one or more patients. At an outset of treatment, the treatment sub-model may input an initial treatment plan of the initial care plan along with any patient data (e.g., diagnosis, parameter data, ect) to obtain a treatment recommendation. The treatment recommendation may include administration of pharmaceuticals or other treatment substances into the patient, administration of a therapy provided by a device or care team member, etc.

According to an aspect, a neural network of the treatment sub-model may be trained to provide recommendations using supervised learning. The supervised learning may use on annotated training data in which patient information is annotated with a treatment plan information. That is, training data may include a patient's diagnosis, medical history, clinical assessment information, device data feeds, and test results, among other forms of relevant patient data. The ground truth may be obtained from the annotated treatment plan information. Based on the patient data inputs and the corresponding treatment plan information-based ground truth, the neural network may be trained to generate recommended treatment plans. For example, large datasets of real-world patient information and corresponding patient monitoring plans may be used to train a neural network of the treatment sub-model. According to an aspect, the sub-model may also be trained based on use feedback. For example, the treatment sub-model may be trained based on information corresponding to whether a care team member accepts, revises, or discards the recommendation or a portion thereof.

An equipment sub-model may include one or more AI models, such as neural networks, trained to recommend a treatment plan based on a patient's diagnosis, medical history, clinical assessment information, device data feeds, test results, output of the patient care model, and facility inventory/availability (e.g., medical device inventory/availability) among other forms of relevant data, for one or more patients. At an outset of treatment, the equipment sub-model may input an initial equipment list of the initial care plan along with any patient and facility data (e.g., diagnosis, parameter data, etc) to obtain an equipment recommendation. The equipment recommendation may include types of devices to use for performing monitoring and treatment and devices for caring for the patient.

According to an aspect, a neural network of the equipment sub-model may be trained to provide recommendations using supervised learning. The supervised learning may use on annotated training data in which patient information is annotated with a treatment plan information. That is, training data may include a patient's diagnosis, medical history, clinical assessment information, device data feeds, test results, among other forms of relevant patient data. The ground truth may be obtained from the annotated treatment plan information. Based on the patient data inputs and the corresponding treatment plan information-based ground truth, the neural network may be trained to generate recommended treatment plans. For example, large datasets of real-world patient information and corresponding patient monitoring plans may be used to train a neural network of the treatment sub-model. According to an aspect, the sub-model may also be trained based on use feedback. For example, the equipment sub-model may be trained based on information corresponding to whether a care team member accepts, revises, or discards the recommendation or a portion thereof.

The patient care model may include additional sub-models trained to assist a care team member in caring for a diagnosed patient. For example, sub-models may be trained to assist with resource allocation and inventory management, as well as assess readmission risk.

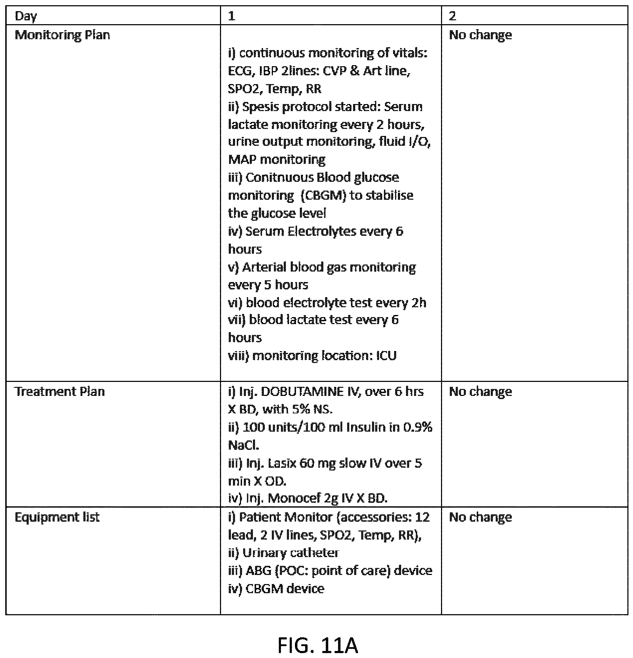

FIGs. 11A-D show an example of outputs of the care assistant, according to an aspect. In this example, the care assistant input diabetic ketoacidosis, congestive cardiac failure, and underlying sepsis as the patient diagnosis, and the following patient information: 1. ECHO impression: low ejection fraction <45 % indicative of congestive heart failure. 2. ECG: abnormal ECG changes: sinus tachycardia, tall R waves with QRS widening, ST changes: flatten T - T wave inversion. 3. blood sugar: random blood sugar test: 220 mg/dl. 4. blood electrolytes: deranged Na, K, Cl: Low potassium, high sodium & chloride. 5. ABG profile: > metabolic acidosis: PH- 7.1, high anion gap, low serum bicarbonate and 6. blood tests: shows high TLC indicative of infection, serum lactate > 3 mmol/liter.

Based on the above inputs, the patient care model may output the initial care plan shown in FIG. 11A. As shown in FIG. 11A, the care plan may include a monitoring plan, a treatment plan, and an equipment list. According to an aspect, information used by the care assistant to obtain the monitoring plan may be generated by the monitoring sub-model, information used by the care assistant to obtain the treatment plan may be generated by the treatment sub-model, and information used by the care assistant to obtain the equipment may be obtained from the equipment sub-model. According to another aspect, the initial care plan may be generated and input by the care team.

As shown in day one of the monitoring plan, parameters to monitor as well as timing corresponding to the monitoring are listed. For example, vitals such as ECG and invasive blood pressure (IVP) are to be continuously monitored, while arterial blood gas (ABG) is monitored every two hours. As shown in day one of the treatment plan, treatments along with their amounts and any necessary timing are listed. As shown in day 1 of the equipment list, recommended equipment to care for the patient are listed. The equipment may include devices for the recommended monitoring (e.g., patient monitor), treatment devices, and other patient care equipment (e.g., urinary catheter).

In addition, the care assistant may recommend a location for the patient. In the example of FIGs. 11A-D, on day 1, the care assistant recommends that the patient is in the intensive care unit (ICU) due to the condition of the patient and the monitoring that is to be performed. As the patient condition improves, the care assistant may recommend transferring the patient to other locations such as step down unit or the patient's home.

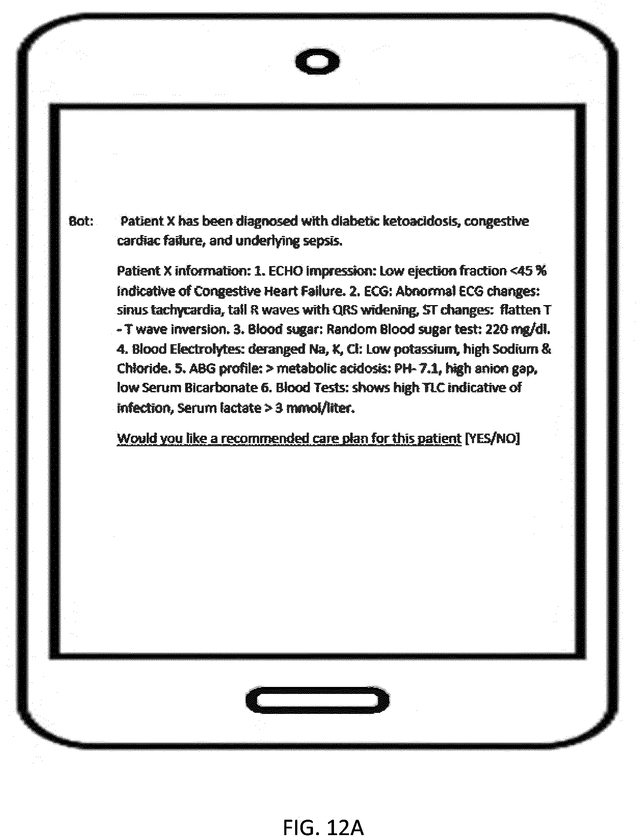

FIGS. 12A-12G show a chatbot interface for the care assistant, according to an aspect. FIGS. 12A-12E show the chatbot outputting the initial care plan, according to an aspect.

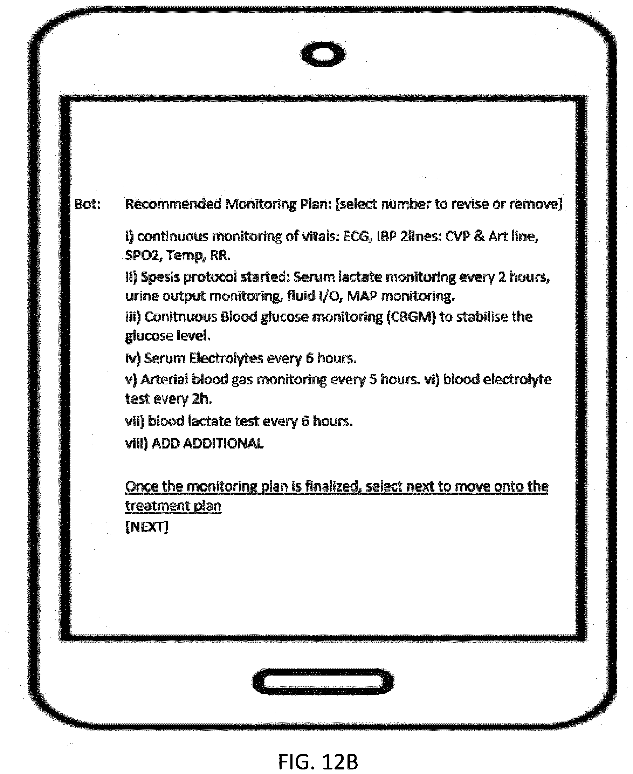

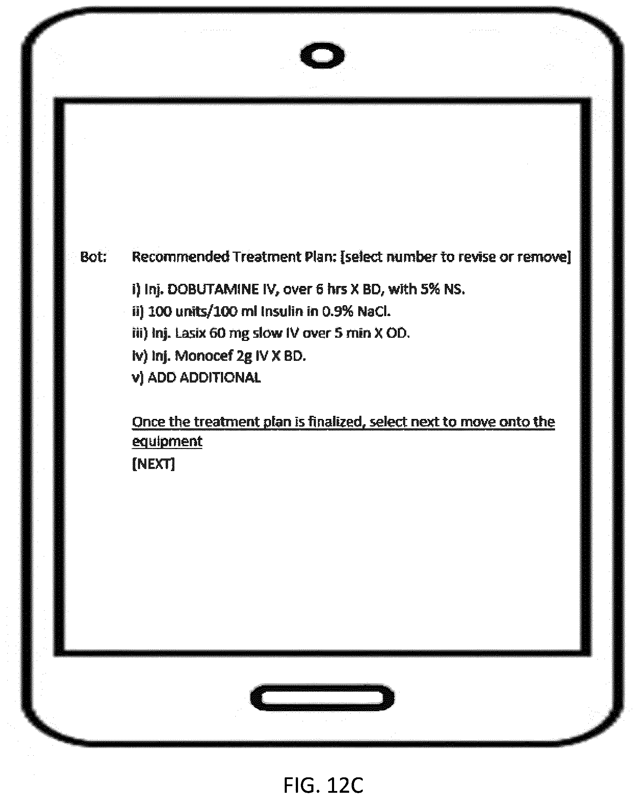

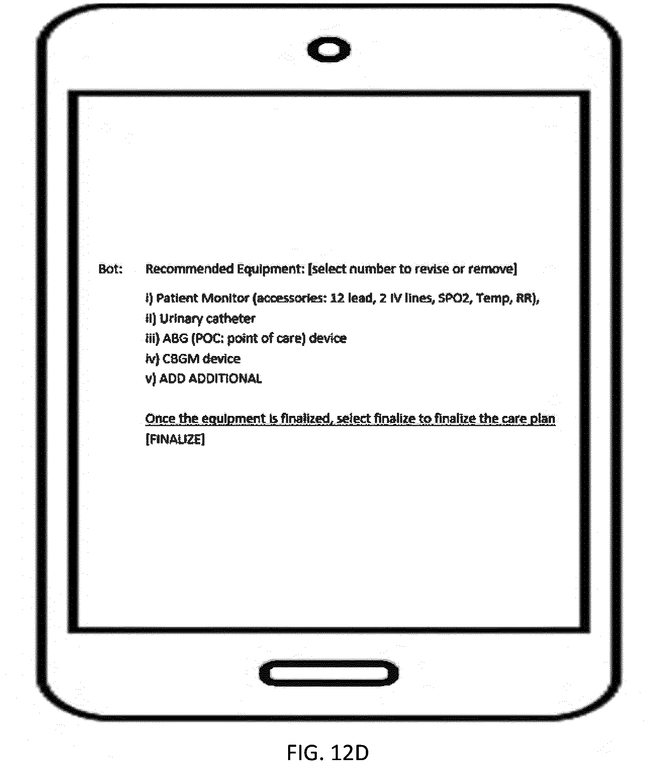

As shown in FIG. 12A, the chatbot may provide the patient diagnosis along with additional patient information. By selecting "YES," a care team member is able to obtain an initial recommended care plan from the care assistant. As shown in FIG. 12B, a recommended monitoring plan of the recommended care plan is displayed for the care provider. The care provider is able to revise, delete, and add to the list through the chatbot. Any additions, deletions, rational, end of case reviews and revisions may be saved for training the patient care model. Once the monitoring list is acceptable for the care team member, the member is able to progress to the treatment plan, shown in FIG. 12C. Similar to the monitoring plan, the care provider is able to revise, delete, and add to the treatment plan through the chatbot. Any additions, deletions, and revisions may be saved for training the patient care model. Once the treatment plan is acceptable for the care team member, the member is able to progress to the equipment, shown in FIG. 12D. Similar to the monitoring and treatment plan, the care provider is able to revise, delete, and add to the equipment list through the chatbot. Any additions, deletions, and revisions may be saved for training the patient care model. Once the care team member finds all of the monitoring, treatment, and equipment plans to be acceptable, the member is able to finalize the care plan.

The finalized care plan may be output by the care assistant to a facility in which the patient is located. For example, the care plan may be output to multiple members of the care team so the monitoring and treatments on the plan can be initiated. According to an aspect, the finalized list may be output to a database so it can be used to further train the patient care model. According to another aspect, the list may be output to an EMR database.

In the example shown in FIGs. 11A-D, a care plan is generated daily based on new patient information received since the last recommendation was generated. For example, every morning at Sam, a new care plan may be generated for the care team member making morning rounds. New patient information may include new monitoring information (e.g., device data feeds), new test results, and new observation input by a care team member. According to another aspect, a new care plan may be generated every time new information is received. For example, the care assistant may generate a new care plan based on receiving new lab results since the results may indicate new treatment and/or monitoring is needed or current treatment and/or monitoring can be stopped.

As shown in FIG. 11A, on day 2, the care assistant may not recommend a change in any of the monitoring plan, the treatment plan, or the equipment. In some instances, the newly generated care recommendation will be the same as the present care plan since the new information would not result in anything being changed. In these cases, since there is no difference between the recommended care plan and the present care plan, the care assistant may not recommend any changes.

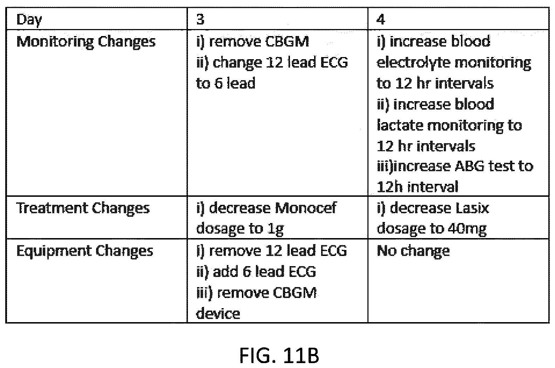

FIG. 11B shows recommendations output on days 3 and 4 of care by the care assistant, according to an aspect.

In the example of FIGS. 11A-11D, after generating the day 2 care recommendation and prior to generating the day 3 care recommendation, the care assistant received information that the patient's blood glucose has stabilized and the patient's ECG has normalized. Based on receiving this information, the care assistant recommends i) removal of continuous blood glucose monitoring (CBGM) and ii) replacing the 12 lead ECG with a 6 lead ECG. Since blood glucose has stabilized, it is no longer necessary to monitor blood glucose. As such, the care assistant recommends removing the blood glucose monitoring to prevent facility resources (e.g., CBGM device) from being used in a wasteful manner. Similarly, since the patient's ECG has normalized and the detail from the 12 lead ECG is no longer necessary for proper care, the care assistant recommends switching to a less expensive 6 lead ECG to save costs. In addition to the changes in monitoring, based on the stabilized glucose and normalized ECG, the care assistant recommends decreasing the dosage of Monocef from 2g to 1g since the patient's condition has improved.

The recommendations, also referred to as care insights, may be obtained by comparing the recommended care plan to the present care plan. Any differences can be output by the care assistant as recommendations or care insights.

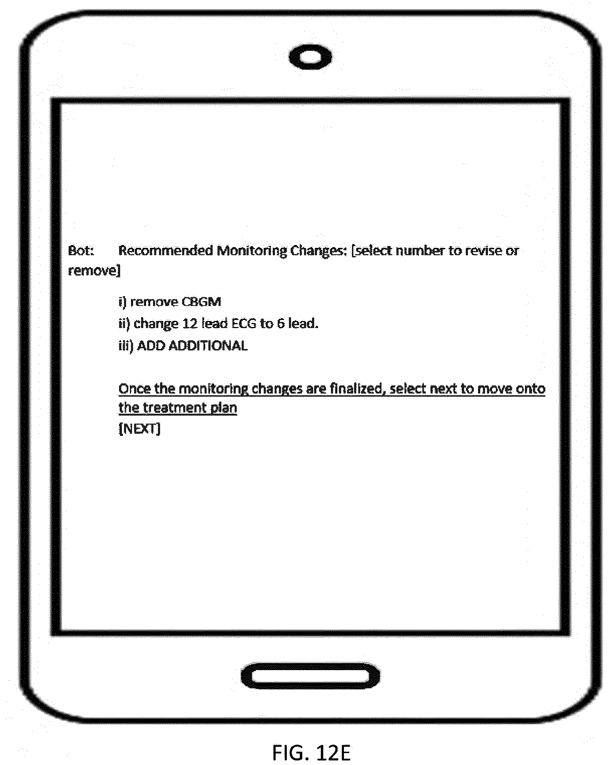

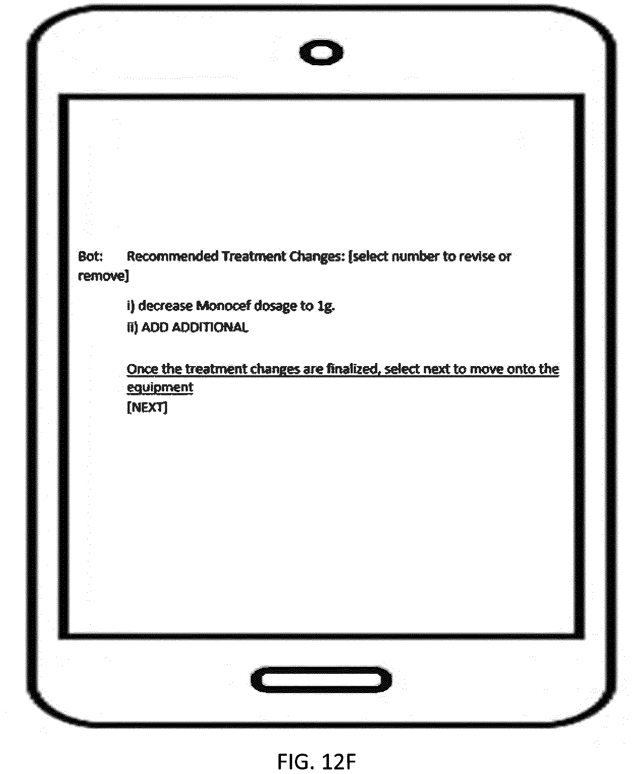

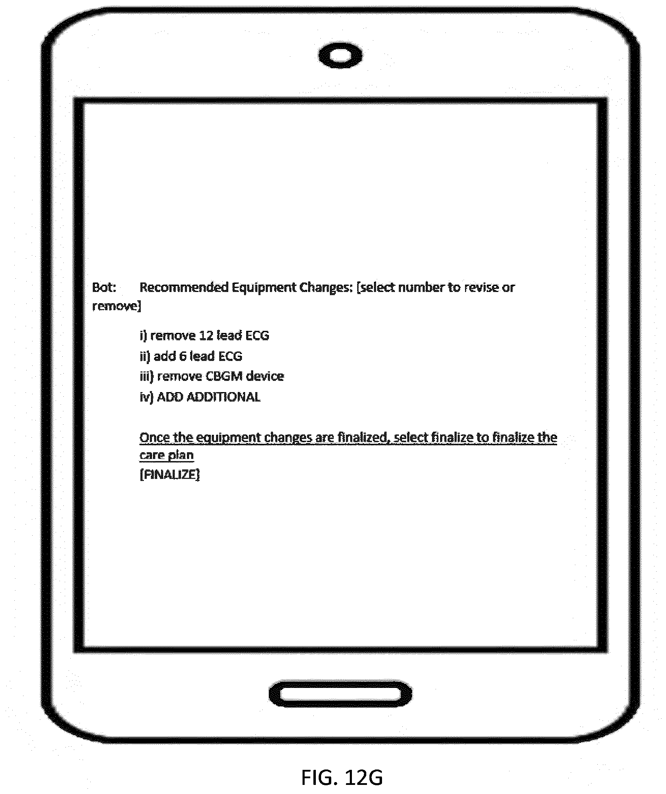

FIGS. 12E-12G show the chatbot outputting changes recommended by the care assistant, according to an aspect. As shown in FIG. 12E, recommended changes to the monitoring plan are displayed for the care provider. The care provider is able to select, revise, and add to the recommended changes through the chatbot. Any additions, revisions, end of case reviews, rationals, and selections may be saved for training the patient care model. Once the monitoring list is acceptable for the care team member, the member is able to progress to the treatment plan, shown in FIG. 12F. Similar to the monitoring plan, the care provider is able to select, revise, and add to recommended changes to the treatment plan through the chatbot. Any additions, revisions, and selections may be saved for training the patient care model. Once the treatment plan is acceptable for the care team member, the member is able to progress to the equipment, shown in FIG. 12G. Similar to the monitoring and treatment plan, the care provider is able to select, revise, and add to the recommended changes to the equipment list through the chatbot. Any additions, revisions, and selections may be saved for training the patient care model. Once the care team member finds all of the monitoring, treatment, and equipment changes to be acceptable, the member is able to finalize the changes to the care plan.

The finalized care plan changes may be output by the care assistant to a facility in which the patient is located. For example, the changes may be output to multiple members of the care team so the monitoring and treatment on the list can be initiated. According to an aspect, the changes may be output to a database so it can be used to further train the care assistant. According to another aspect, the changes may be output to an EMR database.

As shown in FIG. 11B, on day 4 of care, based on receiving information indicating that the patient is improving (e.g., improved vitals, normal test results) the care assistant may recommend increasing the interval between blood tests and may decrease the Lasix dosage. Since these recommended changes do not involve changing any equipment, the care assist may have not recommended any equipment changes.

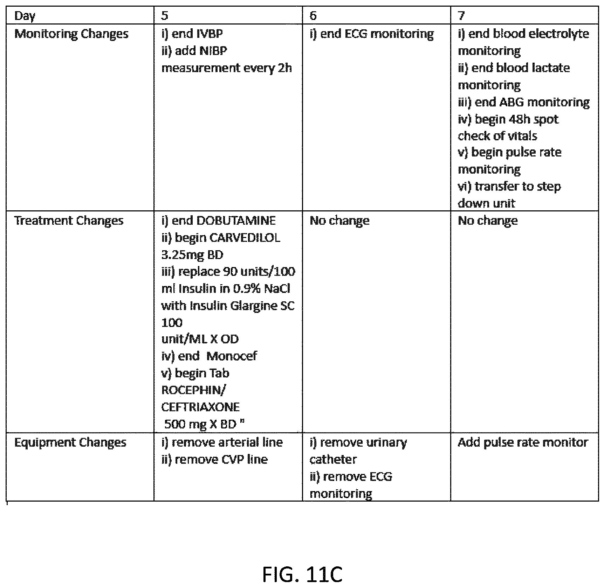

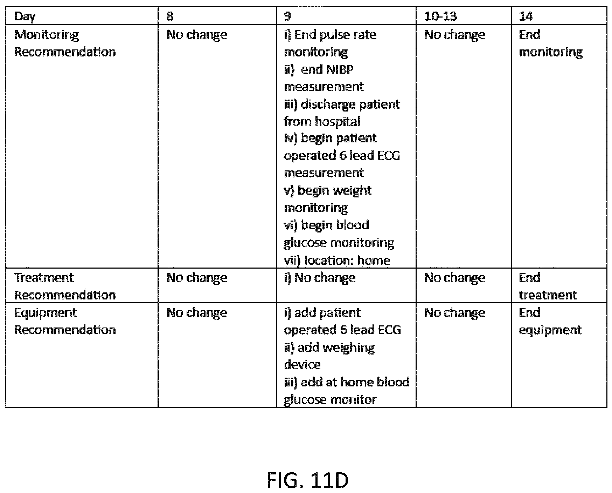

FIG. 11C shows recommended monitoring, treatment, and equipment changes for days 5-7 of care. As shown on day 7, the care assistant recommends transferring the patient to a step-down unit. The care assistant may recommend this transfer because it has received information indicating that the patient's condition has improved. FIG. 11D shown change recommendation for days 8-14. As shown on day 14, the care assistant recommends ending monitoring, treatment, and equipment due to information received by the care assistant indicating that the patient's condition has improved.

As discussed with respect to FIGs. 11A-D, the care assistant may provide recommendations to care team members based on the newly received patient information. The recommendations of the care assistant may change based on new information being received and input into the patient care model to generate recommended changes. For example, when inputting the same initial information into the care assistant as in the example FIGs. 11A-D, the care assistant may output the same care plan. However, based on the patient reacting differently to the treatment, the care assistant may provide different recommendation.

In the example of FIGs. 11A-D, on day 3, the care assistant recommends removal of CBGM and reduction of ECG lead based on receiving new patient information (e.g., information obtained since the previous recommendation was generated) indicating that the patient's blood glucose has stabilized and condition is improving. In an alternative example in which the patient's blood glucose does not stabilize, the care assistant may not recommend ending CBGM.

In the example of FIGs. 11A-D, on day 9, the care assistant recommends discharging the patient from the healthcare facility because the patient's vitals have normalized. In an alternative example in which the patient's vitals normalize on day 5, the care assistant may recommend discharging the patient on day 6.

As discussed above, any recommendation output by the care assistant may be revised or deleted by a care team member through the chatbot. A care team member may also add a monitoring plan, a treatment, and/or equipment to the care plan through the chatbot. These deletions, revisions, and additions may be used by the care assistant when making future recommendations. For example, when a care team member adds a treatment to the care plan, the care assistant may recommend adjustments to the treatment based on newly received patient information. According to another example, when a care team member adds a treatment to the care plan, the care assistant may recommend removing another treatment that is redundant in view of the added treatment or interacts poorly with the added treatment.

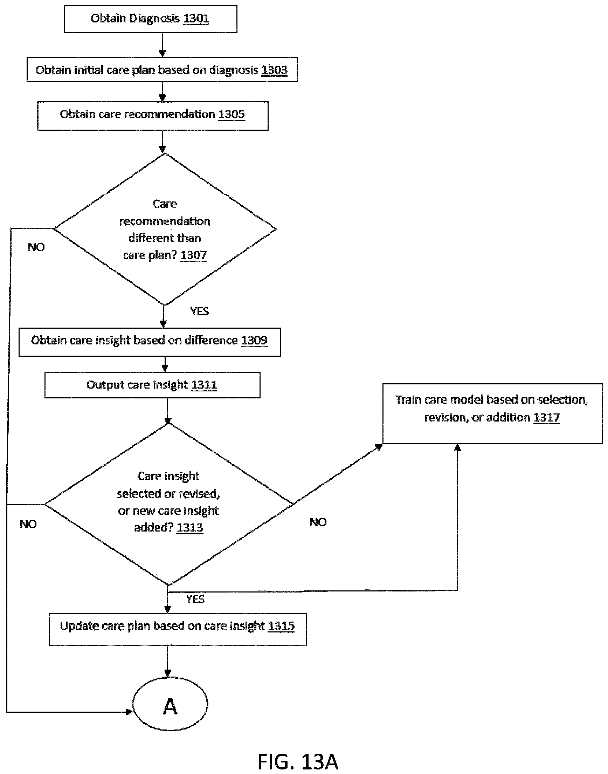

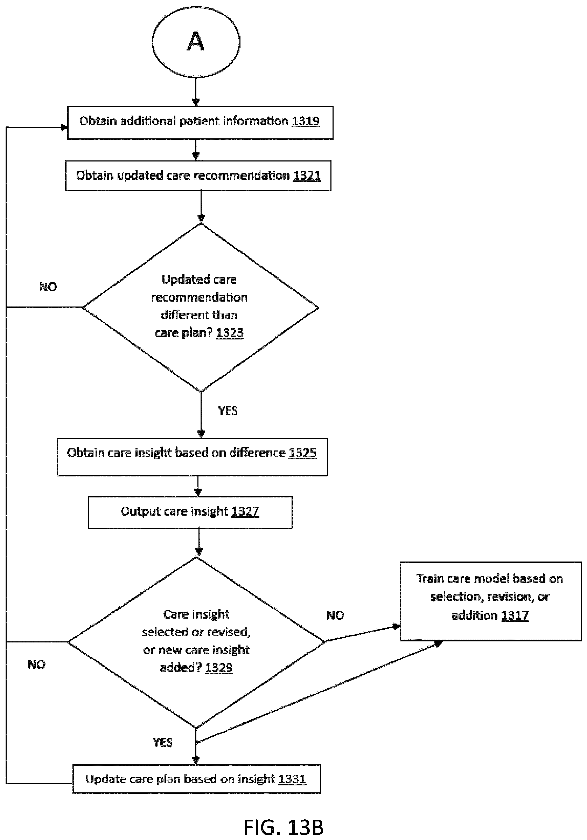

FIGs. 13A and 13B are flowcharts showing a care assistance method 1300, according to an aspect. The method 1300 may be implemented on electronic device 600, in network environment 700, and/or in the environment of FIG. 1. The method 1300 may be implemented using the care assistant. The care assistant may interact with a user via a chatbot, as shown in FIGs 12A-G, or other know user interfaces.

At operation 1301, a diagnosis for a patient may be obtained. According to an aspect, the diagnosis may be obtained using the above-described DAM assistant. A care team member may use the DAM assistant narrow down a list of possible diagnoses to obtain a final diagnosis list. According to another aspect, a diagnosis may be input by a user through the chatbot interface or a standardized form interface.

At operation 1303, a care plan may be obtained based on the diagnosis. According to an aspect, a care plan may be a standardized care plan obtained from a database. According to another aspect, the care plan may be created by a member of the care team. According to another aspect, a standardized care plan may be provided to a care team member and the care team member may revised the standardized care plan to obtain the care plan that is input into the patient care model. According to another aspect, the care plan may be obtained by a trained AI model that inputs a diagnosis as well as other medical information corresponding to the patient, such as patient parameter data from medical device, patient medical history data from an EMR, and lab/test result data. The AI model may be trained based on real world data annotated based on the care plan which acts as the ground truth. The care plan may include a monitoring plan, a treatment plan, and an equipment list.

At operation 1305, a care recommendation may be obtained for the patient using the patient care model. The patient care model may generate a care recommendation based on an input of the diagnosis, medical information of the patient, monitoring data obtained from the patient, and/or inventory information of the facility. For example, the patient care model may input patient parameter data including real time data feeds from sensors measuring the patient, medical history from an electronic medical record, observations made by a care team member, lab/test result information, available equipment at a facility, available beds at a facility, among other relevant information used to obtain a patient care plan.

The care recommendation may include a patient monitoring plan. The patient monitoring plan may include one or more parameters to monitor, as well as timing information for the monitoring. The patient monitoring plan may be obtained from a monitoring sub-model of the patient care model.

The care recommendation may include a treatment plan. The treatment plan may include a medical treatment type, an amount of the type of treatment, and a timing information for the treatment. The treatment plan may be obtained from a treatment sub-model of the patient care model.

The care recommendation may include an equipment list. The equipment list may include a device for monitoring the patient, a device for treating the patient, and/or patient care equipment. The treatment plan may be obtained from an equipment sub-model of the patient care model. According to an aspect, the equipment list sub-model may use inventory information of a facility to determine which equipment to recommend. For example, if 6-lead ECG monitoring is recommended for a patient but the facility does not have available 6-lead ECGs the equipment list sub-model may recommend a 12-lead ECG since the 12 lead will provide the same data as the 6 lead along with additional data that can be discarded.

At operation 1307, the care recommendation may be compared to the care plan to determine if any portions of the care recommendation are different than the care plan. If the care plan and care recommendation are the same, the patient may be adequately cared for and no adjustment to the care plan are necessary, so the method progresses to operation 1319 in which new information is received. If the care recommendation is different than the care plan, the patient may not be adequately cared for, thus adjustments to the care plan may be necessary.

If there is a difference between the care recommendation and the care plan at operation 1307, the method may proceed to operation 1309. At operation 1309, a care insight may be obtained based on the difference between the care recommendation and the care plan. For example, a care plan may include 12-lead ECG monitoring for 24h, blood glucose monitoring for 24h, and blood electrolyte tests every 12h, while a care recommendation may include 12-lead ECG monitoring for 36h, blood glucose monitoring for 24h, blood electrolyte tests every 8h, and SP02 monitoring for 36h. In this case, the care insight may include 12-lead ECG monitoring for 36h, blood electrolyte tests every 8h, and SP02 monitoring for 36h.

At operation 1311, the care insight may be output. The insight may be output to a user to inform the user of recommended changes to the care plan and allow the user to adjust the care plan based on the recommendations. For example, the care insight may be output to a user through a user interface, such as the chatbot shown in FIGs 12A-G. According to an aspect, the care insight may be distributed to one or more devices over a network.

At operation 1313, a user may select or revise a care insight, or add a new care insight, for addition to the care plan. Based on a user selecting, revising, or adding a care insight, the care plan may be revised to include the selected, revised, or added insight at operation 1315. When considering the above example, a care team member may select the 12-lead ECG monitoring for 36h from the care insight. This selection may cause the 12-lease ECG monitoring for 24h of the care plan to be replaced with 12-lead ECG monitoring for 36h. In addition, a care team member may revise the SPO2 monitoring for 36h to 24h. This revised care will then be added to the care plan. If a care team member determines that temperature monitoring for 36h is a necessary care for the patient, the care team member may add temperature monitoring for 36h as a new care insight, which will then be added to the care plan.

Based on a user not selecting, revising, or adding a care insight, the care plan may not be revised and the method may proceed to operation 1319 in FIG. 13B.

At operation 1317, information indicating a user selecting, revising, or adding a care insight may be used to train the patient care model. For example, information indicating a user input selected a recommendation may be used to train the patient care model to more strongly recommend the recommendation in future similar situations. Information indicating a user input revised a recommendation may be used to train the patient care model to recommend the revised recommendation in future similar situations. Information indicating a user did not select a recommendation may be used to train the patient care model to recommend less or not recommend the recommendation in future similar situations. The training may be performed using known methods in the art, such as supervised learning using annotated data.

At operation 1319, additional patient information may be obtained. Additional patient information (also referred to as new information) may include information received after a previous recommendation was generated. The additional information may include medical information of the patient, monitoring data obtained from the patient, and inventory information of the facility. For example, patient monitor data streams and lab results received since the previous care recommendation was generated may be additional information that will be used when generating an updated care recommendation.

At operation 1321, an updated care recommendation may be obtained using the additional information along with the information used when generating the previous care plan. The updated care recommendation may be generated by the patient care model in a similar manner as described in operation 1305. In some cases, the additional information may not result in the updated care recommendation being different than the previous care recommendation. However, in cases where the additional information indicates that the state of the patient has changed, this additional information may cause the patient care model to generate an updated care recommendation that is different than the previously generated care plan.

Generation of the updated care recommendation may be triggered by an event. According to an aspect, an updated care recommendation may be generated every time additional information is received. For example, an updated care recommendation may be generated by that patient care model responsive to receiving new lab results. According to other aspects, an updated care recommendation may be generated at a preset interval. For example, an updated care recommendation may be generated at 5 am every morning so the care team is provided with recommendations based on the most up to date information. According to another aspect, an updated care recommendation may be generated based on a user input. For example, when a care team member is making rounds in a medical facility, the care team member may instruct the care assistant to generate an updated care recommendation.

At operation 1323, the updated care recommendation may be compared to the care plan to determine if any portions of the updated care recommendation are different than the care plan, in a similar manner as operation 1307. Operations 1325, 1327, 1329, and 131 may be performed in a similar manner as operations 1309, 1311, 1313, and 1315, respectively.

If there are no selections, revisions, or additions at operation 1329, the method 1300 may proceed back to operation 1319 in which new information is obtained for generating future recommendation. Similarly, if there are selections, revisions, or additions at operation 1329, the method 1300 may proceed back to operation 1319 once the care plan has been updated at operation 1331.

The care assistance method 1300 may assist a care team when caring for a patient. Care team members are often overworked and working in a hectic environment which can cause in unintentional errors that may result in less than optimal care. A care assistant implementing the care assistance method 1300 by interacting with the care team may prevent unintentional errors that may results in less than optimal care. In addition, care team members may not have the necessary knowledge for proving optimal care. By interacting with the care assistant when caring for patients may increase the quality of care by providing the care team with knowledge they may not have.

According to an aspect of the disclosure, a medical system may include: one or more patient monitoring devices, each patient monitoring device being configured to generate patient monitoring data by monitoring a physiological parameter of a patient; at least one database storing medical information corresponding to the patient; an electronic device including: a power source; a communication interface configured to communicate with the one or more patient monitoring devices and the at least one database over a network; a human-machine interface configured to provide information to a user and obtain information from the user; a memory configured to store instructions; and at least one processor configured to execute the instructions to: obtain, through the communication interface, medical information corresponding to the patient from the at least one database; obtain, through the communication interface, real time or near real time patient monitoring data corresponding to the patient from the one or more patient monitoring devices; generate, by an artificial intelligence (AI) model, a differential diagnosis list for the patient based on at least one of the medical information and the patient monitoring data, the differential diagnosis list including one or more diagnoses and a predicted probability corresponding to each diagnosis, each predicted probability indicating an estimated accuracy of a corresponding diagnosis; provide, through the human-machine interface, the differential diagnosis list and predicted probabilities to the user; and obtain, from the user and through the human-machine interface, user predictions for one or more of the diagnoses of the differential diagnosis list. The AI model includes a first neural network that is trained to generate the predicted probabilities based on previously input user predictions.

According to an aspect of the disclosure, the one or more patient monitoring devices comprises one or more of a blood pressure monitor, a blood oxygen monitor, an electrocardiogram, an electroencephalogram, a temperature monitor, a heart rate monitor, a respiration rate monitor, a carboxyhemoglobin monitor, an end-tidal carbon dioxide monitor, a heart rhythm monitor, a cardiac output monitor, and a heart rate variability monitor.

According to an aspect of the disclosure, the at least one processor may be further configured to: continuously input the real time or near real time patient monitoring data corresponding to the patient into the AI model; and autonomously update the predicted probabilities in response to the patient monitoring data indicating that one or more of the differential diagnosis list and the predicted probabilities should be adjusted.

According to an aspect of the disclosure, the first neural network may be trained to obtain predicted probabilities by: comparing one or more predicted probabilities output by the first neural network to one or more user predictions; and adjusting parameters of the first neural network based on a difference obtained from the comparison.

According to an aspect of the disclosure, the at least one processor may be further configured to: generate one or more recommendations for a first diagnosis of the differential diagnosis list; and generate a recommendation weight for each of the one or more recommendations, the recommendation weight indicating a change in a predicted probability of a diagnosis based on the information corresponding to the recommendation being considered in the corresponding diagnosis.

According to an aspect of the disclosure, the one or more recommendations may include one of an additional parameter to be monitored, a diagnostic test to be performed, and an imaging study to be performed.

According to an aspect of the disclosure, the at least one processor may be further configured to, for a first recommendation of the first diagnosis: generate a predicted probability increase based on the first recommendation indicating that the first diagnosis is accurate; generate a predicted probability decrease based on the first recommendation indicating that the first diagnosis is inaccurate; and generate a weight for the first recommendation by combining the predicted probability increase and the predicted probability decrease.

According to an aspect of the disclosure, the at least one processor may be further configured to autonomously adjust the predicted probabilities in response to information obtained from implementation of a recommendation being input into the AI model.

According to an aspect of the disclosure, the AI model may include two neural networks, the two neural networks including: the first neural network trained to generate the differential diagnosis list; and a second neural network trained to generate the one or more recommendations.

According to an aspect of the disclosure, the at least one processor may be further configured to determine a relative benefit to cost for each recommendation based on a cost of the recommendation and a weight of the recommendation.

According to an aspect of the disclosure, an electronic device may include: a power source; a communication interface configured to communicate with one or more patient monitoring devices and at least one database over a network; a human-machine interface configured to provide information to a user and obtain information from the user; a memory configured to store instructions; and at least one processor configured to execute the instructions to: obtain, through the communication interface, medical information corresponding to the patient from the at least one database; obtain, through the communication interface, real time or near real time patient monitoring data corresponding to the patient from the one or more patient monitoring devices; generate, by an artificial intelligence (AI) model, a differential diagnosis list for the patient based on at least one of the medical information and the patient monitoring data, the differential diagnosis list including one or more diagnoses and a predicted probability corresponding to each diagnosis, each predicted probability indicating an estimated accuracy of a corresponding diagnosis; provide, through the human-machine interface, the differential diagnosis list and predicted probabilities to the user; and obtain, from the user and through the human-machine interface, user predictions for one or more of the diagnoses. The AI model may include a first neural network that is trained to generate the predicted probabilities based on previously input user predictions.

According to an aspect of the disclosure, an electronic device may include a power source; a communication interface configured to communicate with one or more patient monitoring devices and at least one database over a network; a human-machine interface configured to provide information to a user and obtain information from the user; a memory configured to store instructions; and at least one processor configured to execute the instructions to: obtain, through the communication interface, medical information corresponding to the patient from the at least one database; obtain, through the communication interface, real time or near real time patient monitoring data corresponding to the patient from the one or more patient monitoring devices; generate, by an artificial intelligence (AI) model: a differential diagnosis list for the patient based on at least one of the medical information and the patient monitoring data, the differential diagnosis list including one or more diagnoses; a predicted probability corresponding to each diagnosis, each predicted probability indicating an estimated accuracy of a corresponding diagnosis; one or more recommendations for a first diagnosis of the differential diagnosis list; and a recommendation weight for each of the one or more recommendations, the recommendation weight indicating a change in a predicted probability of a diagnosis based on the information corresponding to the recommendation being considered in the corresponding diagnosis; and provide, through the human-machine interface, the differential diagnosis list, the predicted probabilities, the one or more recommendations, and the one or more recommendation weights to the user. The one or more recommendations may include one of an additional parameter to be monitored, a diagnostic test to be performed, and an imaging study to be performed

According to another aspect of the disclosure, the at least one processor may be configured to obtain, from the user and through the human-machine interface, a user prediction for a diagnosis of the differential diagnosis list.

According to another aspect of the disclosure, the at least one processor may be configured to input the user prediction into the AI model to generate one or more of new recommendations and new recommendation weights.

According to another aspect of the disclosure, the at least one processor may be configured to obtain a value of a parameter that is being monitored, the value indicating an amount that the parameter is contributing to a corresponding diagnosis.

According to another aspect of the disclosure, the at least one processor may be further configured to recommend ending monitoring of the parameter that is being monitored based on the value being below a predefined threshold.

According to another aspect of the disclosure, the at least one processor may be configured to: continuously input the real time or near real time patient monitoring data corresponding to the patient into the AI model; and autonomously update the predicted probabilities based on the patent monitoring data.

According to another aspect of the disclosure, the first neural network may be trained to obtain predicted probabilities by: comparing one or more predicted probabilities output by the first neural network to one or more user predictions; and adjusting parameters of the first neural network based on a difference obtained from the comparison.

According to another aspect of the disclosure, the at least one processor is further configured to, for a first recommendation of the first diagnosis: generate a predicted probability increase based on the first recommendation indicating that the first diagnosis is accurate; generate a predicted probability decrease based on the first recommendation indicating that the first diagnosis is inaccurate; and generate the weight for the first recommendation by combining the predicted probability increase and the predicted probability decrease.

According to another aspect of the disclosure, the at least one processor may be configured to autonomously adjust the predicted probabilities in response to information obtained from implementation of a recommendations being input into the AI model.

According to an aspect, a medical system may include one or more patient monitoring devices, each patient monitoring device being configured to generate patient monitoring data by monitoring a physiological parameter of a patient; at least one database storing medical information corresponding to the patient; and an electronic device including a power source; a communication interface configured to communicate with the one or more patient monitoring devices and the at least one database over a network; a human-machine interface configured to provide information to a user and obtain information from the user; a memory configured to store instructions; and at least one processor configured to execute the instructions to: obtain, through the communication interface, a care plan for the patient; obtain a care recommendation by inputting a diagnosis and one or more of medical information corresponding to the patient, monitoring data corresponding to the patient, and medical facility inventory information into a patient care model; compare the care recommendation to the care plan to obtain a care insight, the care insight including recommended care that is not included in the care plan; output, through the human-machine interface, information corresponding to the care insight, and responsive to a user input selecting the care insight through the human-machine interface, revise the care plan based on the care insight.

According to an aspect, a system, may include a power source; a communication interface configured to communicate with one or more patient monitoring devices and at least one database over a network; a human-machine interface configured to provide information to a user and obtain information from the user; a memory configured to store instructions; and at least one processor configured to execute the instructions to: obtain, through the communication interface, a care plan for a patient; obtain a care recommendation by inputting a diagnosis and one or more of medical information corresponding to the patient, monitoring data corresponding to the patient, and medical facility inventory information into a patient care model; compare the care recommendation to the care plan to obtain a care insight, the care insight including recommended care that is not included in the care plan; output, through the human-machine interface, information corresponding to the care insight, and responsive to a user input selecting the care insight through the human-machine interface, revise the care plan based on the care insight.

According to an aspect, the at least one processor may be configured to execute the instructions to obtain the care recommendation by obtaining a patient monitoring plan from the patient care model.

According to an aspect, the patient monitoring plan may include a parameter to monitor and timing information for monitoring the parameter.

According to an aspect, the at least one processor may be configured to execute the instructions to obtain the patient monitoring plan from a monitoring sub-model of the patient care model, the monitoring sub-model being trained based on previous user inputs.

According to an aspect, the at least one processor may be configured to execute the instructions to obtain the care recommendation by obtaining a treatment plan from the patient care model.

According to an aspect, the treatment plan may include a medical treatment type, an amount of the type of treatment, and a timing information for the treatment.

According to an aspect, the at least one processor may be configured to execute the instructions to obtain the treatment plan from a treatment sub-model of the patient care model, the treatment sub-model being trained based on previous user inputs.

According to an aspect, the at least one processor may be configured to execute the instructions to obtain the care recommendation by obtaining an equipment list from the patient care model.

According to an aspect, the equipment list may include at least one of a device for monitoring the patient, a device for treating the patient, and patient care equipment.

According to an aspect, the at least one processor is configured to execute the instructions to obtain the equipment list from an equipment sub-model of the patient care model, the equipment sub-model being trained based on previous user inputs.

According to an aspect, the at least one processor may be configured to execute the instructions to, based on receiving additional medical information and/or additional patient monitoring data: obtain an updated care recommendation by inputting the additional medical information and/or additional patient monitoring data into the patient care model; compare the updated care recommendation to the care plan to obtain an updated care insight, the updated care insight including recommended care that is not included in the care plan; output information corresponding to the updated care insight, and responsive to a user input selecting the updated care insight of the updated care recommendation, revise the care plan based on the updated care insight.

According to an aspect, the at least one processor may be configured to execute the instructions to, at a preset time interval: obtain an updated care recommendation by inputting additional medical information and/or additional patient monitoring data into the patient care model, the additional medical information and/or additional patient monitoring data being created after the care recommendation was obtained; compare the updated care recommendation to the care plan to obtain an updated care insight, the updated care insight including recommended care that is not included in the care plan; output information corresponding to the updated care insight, and responsive to a user input selecting the updated care insight of the updated care recommendation, revise the care plan based on the updated care insight.

According to an aspect, the at least one processor may be configured to execute the instructions to: generate, by a differential diagnosis model, a differential diagnosis list for the patient, the differential diagnosis list being obtained by inputting least one of diagnostic medical information corresponding to the patient and diagnostic monitoring data corresponding to the patient into the differential diagnosis model, the differential diagnosis list including one or more diagnoses; and obtaining the diagnosis from the differential diagnosis model based on a user selection.

According to an aspect, the user input selecting the care insight comprises a user input revising the care insight.

According to an aspect, a method of patient care assistance may include: obtaining a care plan for a patient; obtaining a care recommendation by inputting a diagnosis and one or more of medical information corresponding to the patient, monitoring data corresponding to the patient, and medical facility inventory information into a patient care model; comparing the care recommendation to the care plan to obtain a care insight, the care insight including recommended care that is not included in the care plan; outputting information corresponding to the care insight, and responsive to a user input selecting the care insight through the human-machine interface, revising the care plan based on the care insight.

According to an aspect, the obtaining the care recommendation comprises obtaining a patient monitoring plan, a treatment plan, and an equipment plan from the patient care model.

According to an aspect, the method may further include obtaining a user input for each of the patient monitoring plan, the treatment plan, and the equipment plan.

According to an aspect, the method may further include one or more of i) responsive to receiving additional medical information and/or additional patient monitoring data and ii) at a preset time interval: obtaining an updated care recommendation by inputting the additional medical information and/or additional patient monitoring data into the patient care model; comparing the updated care recommendation to the care plan to obtain an updated care insight, the updated care insight including recommended care that is not included in the care plan; outputting information corresponding to the updated care insight, and responsive to a user input selecting the updated care insight of the updated care recommendation, revise the care plan based on the updated care insight.

According to an aspect, the monitoring data may include data from multiple real time or near real time data feeds produced by multiple sources.

According to an aspect, the method may include obtaining the medical information comprising obtaining medical data from multiple sources and standardizing the medical data for input into the patient care model. For example, different medical devices may provide data in different formats. As such, these different formats may be standardized for input into the care model.

According to an aspect, the method may further include obtaining real-time or near-real time monitoring data from multiple sources and standardizing the monitoring data in real time or near real time for input into the patient care model.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A medical system comprising:
one or more patient monitoring devices, each patient monitoring device being configured to generate patient monitoring data by monitoring a physiological parameter of a patient;
at least one database storing medical information corresponding to the patient; and
an electronic device comprising:
a power source;
a communication interface configured to communicate with the one or more patient monitoring devices and the at least one database over a network;
a human-machine interface configured to provide information to a user and obtain information from the user;
a memory configured to store instructions; and
at least one processor configured to execute the instructions to:
obtain, through the communication interface, a care plan for the patient;
obtain a care recommendation by inputting a diagnosis and one or more of medical information corresponding to the patient, monitoring data corresponding to the patient, and medical facility inventory information into a patient care model;
compare the care recommendation to the care plan to obtain a care insight, the care insight including recommended care that is not included in the care plan or recommended removal of care that is included in the care plan;
output, through the human-machine interface, information corresponding to the care insight, and
responsive to a user input selecting the care insight through the human-machine interface, revise the care plan based on the care insight.

2. A system, comprising:
a power source;
a communication interface configured to communicate with one or more patient monitoring devices and at least one database over a network;
a human-machine interface configured to provide information to a user and obtain information from the user;
a memory configured to store instructions; and
at least one processor configured to execute the instructions to:
obtain, through the communication interface, a care plan for a patient;
obtain a care recommendation by inputting a diagnosis and one or more of medical information corresponding to the patient, monitoring data corresponding to the patient, and medical facility inventory information into a patient care model;
compare the care recommendation to the care plan to obtain a care insight, the care insight including recommended care that is not included in the care plan or recommended removal of care that is included in the care plan;
output, through the human-machine interface, information corresponding to the care insight, and
responsive to a user input selecting the care insight through the human-machine interface, revise the care plan based on the care insight.

3. The system of claim 2, wherein the at least one processor is configured to execute the instructions to obtain the care recommendation by obtaining a patient monitoring plan from the patient care model, and wherein the patient monitoring plan comprises a parameter to monitor and timing information for monitoring the parameter.

4. The system of claim 3, wherein the at least one processor is configured to execute the instructions to obtain the patient monitoring plan from a monitoring sub-model of the patient care model, the monitoring sub-model being trained based on previous user inputs.

5. The system of claim 2, wherein the at least one processor is configured to execute the instructions to obtain the care recommendation by obtaining a treatment plan from the patient care model, and wherein the treatment plan comprises a medical treatment type, an amount of the type of treatment, and a timing information for the treatment.

6. The system of claim 5, wherein the at least one processor is configured to execute the instructions to obtain the treatment plan from a treatment sub-model of the patient care model, the treatment sub-model being trained based on previous user inputs.

7. The system of claim 2, wherein the at least one processor is configured to execute the instructions to obtain the care recommendation by obtaining an equipment list from the patient care model, and wherein the equipment list comprises at least one of a device for monitoring the patient, a device for treating the patient, and patient care equipment, and wherein the at least one processor is configured to execute the instructions to obtain the equipment list from an equipment sub-model of the patient care model, the equipment sub-model being trained based on previous user inputs.

8. The system of claim 2, wherein the at least one processor is configured to execute the instructions to, based on receiving additional medical information and/or additional patient monitoring data:
obtain an updated care recommendation by inputting the additional medical information and/or additional patient monitoring data into the patient care model;
compare the updated care recommendation to the care plan to obtain an updated care insight, the updated care insight including recommended care that is not included in the care plan;
output information corresponding to the updated care insight, and
responsive to a user input selecting the updated care insight of the updated care recommendation, revise the care plan based on the updated care insight.

9. The system of claim 2, wherein the at least one processor is configured to execute the instructions to, at a preset time interval:
obtain an updated care recommendation by inputting additional medical information and/or additional patient monitoring data into the patient care model, the additional medical information and/or additional patient monitoring data being created after the care recommendation was obtained;
compare the updated care recommendation to the care plan to obtain an updated care insight, the updated care insight including recommended care that is not included in the care plan;
output information corresponding to the updated care insight, and responsive to a user input selecting the updated care insight of the updated care recommendation, revise the care plan based on the updated care insight.

10. The system of claim 2, wherein the at least one processor is configured to execute the instructions to:
generate, by a differential diagnosis model, a differential diagnosis list for the patient, the differential diagnosis list being obtained by inputting least one of diagnostic medical information corresponding to the patient and diagnostic monitoring data corresponding to the patient into the differential diagnosis model, the differential diagnosis list including one or more diagnoses; and
obtaining the diagnosis from the differential diagnosis model based on a user selection, and
wherein the user input selecting the care insight comprises a user input revising the care insight.

11. A method of patient care assistance, comprising:
obtaining a care plan for a patient;
obtaining a care recommendation by inputting a diagnosis and one or more of medical information corresponding to the patient, monitoring data corresponding to the patient, and medical facility inventory information into a patient care model;
comparing the care recommendation to the care plan to obtain a care insight, the care insight including recommended care that is not included in the care plan or recommended removal of care that is included in the care plan;
outputting information corresponding to the care insight, and
responsive to a user input selecting the care insight through the human-machine interface, revising the care plan based on the care insight.

12. The method of claim 11, wherein the obtaining the care recommendation comprises obtaining a patient monitoring plan, a treatment plan, and an equipment plan from the patient care model.

13. The method of claim 12, further comprising obtaining a user input for each of the patient monitoring plan, the treatment plan, and the equipment plan.

14. The method of claim 11, further comprising, one or more of i) responsive to receiving additional medical information and/or additional patient monitoring data and ii) at a preset time interval:
obtaining an updated care recommendation by inputting the additional medical information and/or additional patient monitoring data into the patient care model;
comparing the updated care recommendation to the care plan to obtain an updated care insight, the updated care insight including recommended care that is not included in the care plan;
outputting information corresponding to the updated care insight, and responsive to a user input selecting the updated care insight of the updated care recommendation, revise the care plan based on the updated care insight.

15. The method of claim 11, wherein the monitoring data comprises data from multiple real time or near real time data feeds produced by multiple sources.
